(19) Europäisches Patentamt
European Patent Office
Office européen des brevets

(11) Numéro de publication : **0 053 964**
**B1**

(12) # FASCICULE DE BREVET EUROPÉEN

(45) Date de publication du fascicule du brevet : **07.05.86**

(51) Int. Cl.⁴ : **C 07 D401/06**, A 61 K 31/47

(21) Numéro de dépôt : **81401858.6**

(22) Date de dépôt : **24.11.81**

(54) **Médicaments à base de dérivés de (quinolyl-4)-1 éthanol ou propanol, nouveaux dérivés de (quinolyl-4)-1 éthanol ou propanol et procédés pour leur préparation.**

(30) Priorité : **05.12.80 FR 8025829**

(43) Date de publication de la demande : **16.06.82 Bulletin 82/24**

(45) Mention de la délivrance du brevet : **07.05.86 Bulletin 86/19**

(84) Etats contractants désignés : **BE CH DE FR GB IT LI LU NL SE**

(56) Documents cités :
**EP-A- 0 030 044**
**EP-A- 0 035 819**
**EP-A- 0 035 820**
**DE-C- 330 813**
**NL-A- 7 908 030**
**BRITISH JOURNAL OF PHARMACOLOGY AND CHEMOTHERAPY, vol. 1, 1946, LONDRES (GB), G.S. DAWES: "Synthetic substitutes for quinidine", pages 90-111**
**JOURNAL OF THE AMERICAN CHEMICAL SOCIETY, vol. 44, no. 5, mai 1922, WASHINGTON (US), M. HEIDELBERGER et al.: "Syntheses in the cinchona series. X. Dihydrocinchonicinol and the dihydroquinicinols", pages 1098-1107**
**PROCEEDINGS OF THE ROYAL SOCIETY, B125, 1938, LONDRES (GB), A.D. AINLEY et al.: "Antiplasmodial action and chemical constitution. Part II. Some simple synthetic analogues of quinine and cinchonine", pages 60-92**

(73) Titulaire : **PHARMUKA LABORATOIRES**
**35 Quai du Moulin de Cage**
**F-92231 Gennevilliers (FR)**

(72) Inventeur : **Gueremy, Claude Georges Alexandre**
**3, rue Daumesnil**
**F-78800 Houilles (FR)**
Inventeur : **Mestre, Michel Achille Pierre**
**15, rue Alasseur**
**F-75015 Paris (FR)**
Inventeur : **Renault, Christian Louis Albert**
**10 Allée Réaumur**
**F-95150 Taverny (FR)**

(74) Mandataire : **Gaumont, Robert et al**
**RHONE-POULENC RECHERCHES Service Brevets**
**Pharma 25, Quai Paul Doumer**
**F-92408 Courbevoie Cedex (FR)**

**EP 0 053 964 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet de nouveaux médicaments, particulièrement utiles comme antiarythmiques, qui contiennent, en tant que substance active, un composé répondant à la formule :

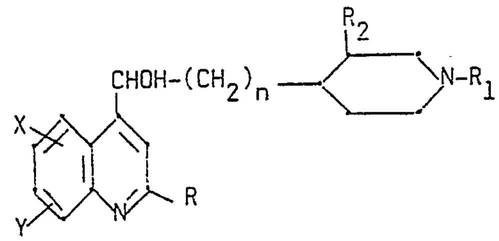

(I)

dans laquelle

a) soit n est égal à 1, X et Y qui sont identiques ou différents, sont fixés en position 5, 6, 7 ou 8 sur le cycle de la quinoléine et représentent chacun l'atome d'hydrogène ou un groupe alcoxy comportant 1 à 3 atomes de carbone, R représente l'atome d'hydrogène, un groupe cycloalkyle ayant 3 à 8 atomes de carbone, un groupe alkyle comportant 1 à 4 atomes de carbone, un groupe phényle ou un groupe phényle substitué par un groupe alcoxy ayant 1 à 4 atomes de carbone, $R_1$ représente l'atome d'hydrogène, un groupe alkyle comportant 1 à 4 atomes de carbone ou un groupe phénylalkyle dont la partie alkyle comporte 1 à 3 atomes de carbone, $R_2$ représente l'atome d'hydrogène, un groupe alkyle comportant 1 ou 2 atomes de carbone ou un groupe alcényle comportant 2 à 4 atomes de carbone,

b) soit n est égal à 2, $R_2$ représente l'atome d'hydrogène ou un groupe alcényle comportant 2 à 4 atomes de carbone, X, Y, R et $R_1$ ont les significations mentionnées ci-dessus en a),

c) soit n est égal à 2, $R_2$ représente un groupe alkyle comportant 1 ou 2 atomes de carbone, R représente un groupe cycloalkyle ayant 3 à 8 atomes de carbone, un groupe phényle ou un groupe phényle substitué par un groupe alcoxy ayant 1 à 4 atomes de carbone, X, Y et $R_1$ ont les significations mentionnées ci-dessus en a),

d) soit n est égal à 2, $R_2$ représente un groupe alkyle comportant 1 ou 2 atomes de carbone, R représente l'atome d'hydrogène, $R_1$ représente l'atome d'hydrogène ou un groupe phénylalkyle dont la partie alkyle comporte 1 à 3 atomes de carbone, X et Y ont les significations mentionnées ci-dessus en a),

e) soit n est égal à 2, $R_2$ représente un groupe alkyle comportant 1 ou 2 atomes de carbone, R représente un groupe alkyle comportant 1 à 4 atomes de carbone, $R_1$ représente un groupe phénylalkyle dont la partie alkyle comporte 1 à 3 atomes de carbone, X et Y ont les significations mentionnées ci-dessus en a),

ou un mélange de composés stéréoisomères répondant à la formule (I) ou un sel d'un tel composé ou mélange de composés stéréoisomères avec un acide pharmaceutiquement acceptable.

Dans la formule (I) ci-dessus, Y est de préférence un atome d'hydrogène, X est de préférence un atome d'hydrogène ou un groupe méthoxy, R est de préférence un atome d'hydrogène ou un groupe phényle ou tertiobutyle, $R_1$ est de préférence un atome d'hydrogène et $R_2$ est de préférence un atome d'hydrogène ou un groupe éthyle ou éthényle.

Lorsque $R_2$ représente l'atome d'hydrogène, la molécule des composés de formule (I) comporte un atome de carbone asymétrique (atome de carbone portant le groupe OH) et donc, pour une signification donnée de X, Y, R, $R_1$, $R_2$ et n, il y a un racémique et deux énantiomères correspondant à la formule plane (I). Lorsque $R_2$ ne représente pas l'atome d'hydrogène, la molécule des composés de formule (I) comporte 3 atomes de carbone asymétriques et donc, pour une signification donnée de X, Y, R, $R_1$, $R_2$ et n, il y a 8 stéréoisomères correspondant à la formule plane (I), stéréoisomères dont les formules spatiales correspondent aux combinaisons 3 à 3 des configurations rectus (R) ou sinister (S) de chaque centre d'asymétrie. Les médicaments selon l'invention peuvent contenir, en tant que substance active, aussi bien un mélange de composés stéréoisomères répondant à la formule (I) qu'un isomère pur répondant à la formule (I).

Il est connu (cf. M. HEIDELBERGER et W. A. JACOBS, J. Am. Chem. Soc. *44*, 1098-1107, (1922) ; brevet allemand n° 330 813) des produits, qui sont probablement des mélanges d'isomères, répondant à la formule :

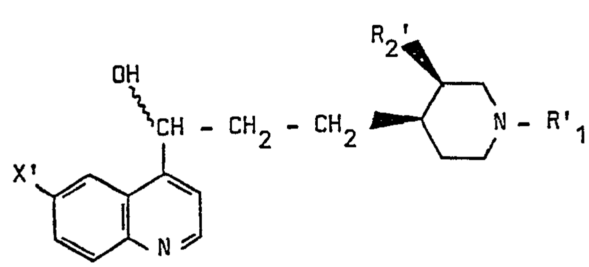

(I bis)

dans laquelle X' est un atome d'hydrogène ou un groupe méthoxy ou éthoxy, $R'_1$ est un atome d'hydrogène ou un groupe méthyle ou éthyle et $R'_2$ est un groupe éthyle ou éthényle, mais aucune propriété pharmacologique ou application thérapeutique n'a été signalée jusqu'ici pour ces produits.

Selon G.S. DAWES (Brit. J. Pharmacol., 1946, 1, 90-111), le composé de formule :

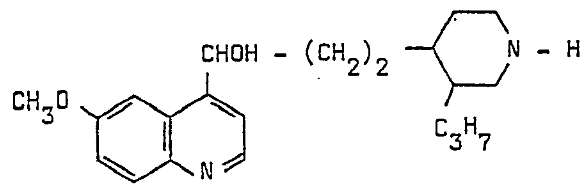

$,HNO_3$

agirait sur l'aptitude de l'oreillette isolée de lapin à suivre les stimulations électriques causées par une bobine à induction, mais le poids moléculaire (391) indiqué par cet auteur pour le composé ci-dessus est incompatible avec la formule, de sorte qu'il y a une incertitude sur la structure réelle du composé testé.

Il a maintenant été trouvé, conformément à la présente invention, que les composés de formule (I) possèdent des propriétés pharmacologiques remarquables qui permettent de les utiliser comme ingrédient actif de médicaments.

Les composés, racémiques, énantiomères ou stéréoisomères, répondant à la formule :

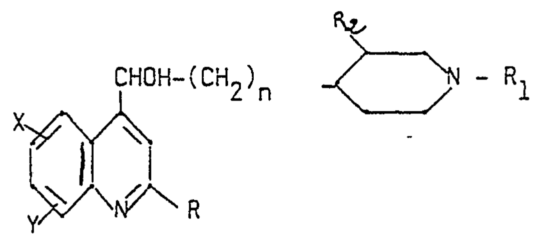

(Ia)

dans laquelle X, Y, R, $R_1$ et $R_2$ ont la même signification que dans la formule (I) et n = 1 sont nouveaux et font partie en tant que tels de l'invention.

Les composés, racémiques, énantiomères ou stéréoisomères, répondant à la formule :

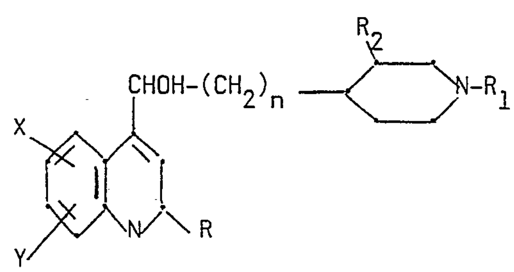

(Ib)

dans laquelle n est égal à 2, X et Y qui sont identiques ou différents, sont fixés en position 5, 6, 7 ou 8 sur le cycle de la quinoléine et représentent chacun l'atome d'hydrogène ou un groupe alcoxy comportant 1 à 3 atomes de carbone et

a) soit $R_2$ est un groupe alcényle comportant 2 à 4 atomes de carbone, R représente l'atome d'hydrogène et $R_1$ représente un groupe phénylalkyle dont la partie alkyle comporte 1 à 3 atomes de carbone,

b) soit $R_2$ est un groupe alcényle comportant 2 à 4 atomes de carbone, R représente un groupe cycloalkyle ayant 3 à 8 atomes de carbone, un groupe alkyle comportant 1 à 4 atomes de carbone, un groupe phényle éventuellement substitué par un groupe alcoxy ayant 1 à 4 atomes de carbone et $R_1$ représente l'atome d'hydrogène, un groupe alkyle comportant 1 à 4 atomes de carbone ou un groupe phénylalkyle dont la partie alkyle comporte 1 à 3 atomes de carbone,

c) soit $R_2$ est un groupe alkyle comportant 1 ou 2 atomes de carbone, R représente l'atome d'hydrogène et $R_1$ représente un groupe phénylalkyle dont la partie alkyle comporte 1 à 3 atomes de carbone,

d) soit $R_2$ représente un groupe alkyle comportant 1 ou 2 atomes de carbone, R représente un groupe alkyle comportant 1 à 4 atomes de carbone et $R_1$ représente un groupe phénylalkyle dont la partie alkyle comporte 1 à 3 atomes de carbone.

e) soit $R_2$ est un groupe alkyle comportant 1 à 2 atomes de carbone, R représente un groupe cycloalkyle ayant 3 à 8 atomes de carbone, un groupe phényle éventuellement substitué par un groupe alcoxy ayant 1 à 4 atomes de carbone, $R_1$ représente un atome d'hydrogène, un groupe alkyle comportant 1 à 4 atomes de carbone ou un groupe phénylalkyle dont la partie alkyle comporte 1 à 3 atomes de carbone sont nouveaux et font partie en tant que tels de l'invention.

Les composés, racémiques ou énantiomères, répondant à la formule :

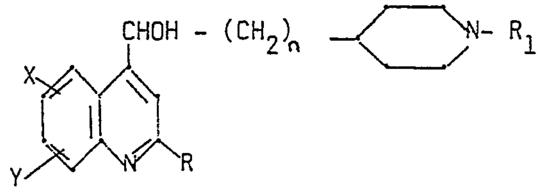

(Ic)

dans laquelle n est égal à 2, X et Y qui sont identiques ou différents, sont fixés en position 5, 6, 7 ou 8 sur le cycle de la quinoléine et représentent chacun l'atome d'hydrogène ou un groupe alcoxy comportant 1 à 3 atomes de carbone, R représente l'atome d'hydrogène, un groupe cycloalkyle ayant 3 à 8 atomes de carbone, un groupe alkyle comportant 1 à 4 atomes de carbone, un groupe phényle ou un groupe phényle substitué par un groupe alcoxy ayant 1 à 4 atomes de carbone, $R_1$ représente l'atome d'hydrogène, un groupe alkyle comportant 1 à 4 atomes de carbone ou un groupe phénylalkyle dont la partie alkyle comporte 1 à 3 atomes de carbone sont nouveaux et font partie en tant que tels de l'invention.

Les composés de formule (I) peuvent être préparés par réduction des cétones de formule :

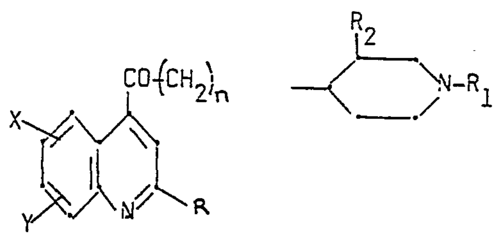

(II)

dans laquelle X, Y, R, $R_1$, $R_2$ et n ont la même signification que dans la formule (I).

Pour effectuer cette réduction on utilise des procédés, connus en soi, qui permettent de transformer une cétone en alcool. Une méthode avantageuse, applicable dans tous les cas, consiste à utiliser comme agent de réduction un hydrure métallique réducteur tel que ceux mentionnés dans « Complex hydrides and related reducing agents in organic synthesis » (Andor HAJOS, Elsevier Scientific Publishing Company, Amsterdam, Oxford, New-York 1979). Parmi les agents réducteurs les plus courants on peut citer les borohydrures de métaux alcalins tels que le borohydrure de sodium et le borohydrure de potassium, qui sont utilisés à la température ambiante au sein d'un solvant tel qu'un alcool (par exemple le méthanol ou l'éthanol), un mélange eau-alcool ou le tétrahydrofuranne, et l'hydrure d'aluminium et de lithium, qui est utilisé au sein d'un solvant inerte tel que l'éther, le tétrahydrofuranne ou un hydrocarbure, à une température comprise entre 0 °C et la température d'ébullition du solvant.

Lorsque $R_2$ est l'atome d'hydrogène, la réduction des cétones de formule (II) fournit le racémique. Lorsque $R_2$ n'est pas l'atome d'hydrogène, la réduction des cétones de formule (II) fournit un mélange de composés diastéréoisomères, racémiques ou optiquement actifs selon que la cétone de départ est racémique ou optiquement active. Les diastéréoisomères purs peuvent être isolés à partir du mélange par des méthodes classiques telles que chromatographie, cristallisation fractionnée, formation de sels et régénération de la base, etc...

Une variante du procédé de réduction ci-dessus consiste à effectuer la réduction en présence d'un composé optiquement actif, capable de complexer l'hydrure réducteur utilisé, par exemple un α-amino-acide (cf. J.D. MORRISON et H.S. MOSHER, Asymetric Organic reactions, Prentice Hall Englewood Cliffs N.J. (1972) ; J.W. APSIMON et R.P. SEGUIN, Tetrahedron, 1979, *35*, 2797 ; J.C. FIAUD, Stereochemistry Fundamentals and Methods, Vol. 3,95, edited by H.B. KAGAN, Georg Thieme Publishers Stuttgart, 1977 ; N. UMINO, Chem. Pharm. Bull., 1979, *27*, 1479). Dans ces conditions on obtient un produit de réduction contenant en quantité prépondérante un énantiomère (si $R_2 = H$) ou un diastéréoisomère (si $R_2 \neq H$), dont l'atome de carbone portant le groupe OH a une configuration déterminée. Par exemple la L-proline induit la formation prépondérante d'un alcool dans lequel l'atome de carbone porteur du groupe OH a la configuration sinister (S) et la D-proline induit la formation prépondérante d'un alcool dans lequel l'atome de carbone porteur du groupe OH a la configuration rectus (R). Cette variante est avantageuse dans le cas où la cétone de départ de formule (II) est optiquement active. On obtient alors un produit de réduction contenant essentiellement un diastéréoisomère déterminé, optiquement actif, qui est facilement isolable par les techniques indiquées plus haut.

Les composés de formule (I) pour lesquels $R_1$ ne représente pas un groupe benzyle et $R_2$ représente un groupe alkyle ayant 2 atomes de carbone peuvent aussi être préparés par hydrogénation catalytique des cétones correspondantes de formule (II) pour lesquelles $R_2$ représente un groupe alcényle ayant 2 atomes de carbone. Dans ce cas on effectue à la fois la réduction du groupe CO en groupe CHOH et l'hydrogénation du groupe alcényle en groupe alkyle. On opère généralement au voisinage de la température ambiante, sous une pression d'hydrogène voisine de la pression atmosphérique, la cétone

de départ (sous forme de la base libre ou de l'un de ses sels) étant au sein d'un solvant inerte tel qu'un alcool (par exemple le méthanol ou l'éthanol), un mélange eau-alcool ou un acide (par exemple l'acide acétique). Comme catalyseurs on peut citer le palladium, le rhodium, le ruthénium, le platine et le nickel.

Les composés de formule (I) pour lesquels $R_1$ est l'atome d'hydrogène et $R_2$ le groupe éthényle et pour lesquels l'atome de carbone porteur du groupe éthényle a une configuration donnée, rectus (R) ou sinister (S), peuvent aussi être préparés par chauffage, à température supérieure à 50 °C, au sein d'un solvant protique ou d'un mélange de solvants protiques, en présence de formaldéhyde, des composés de formule (I) correspondants pour lesquels $R_1$ est l'atome d'hydrogène, $R_2$ est le groupe éthényle et l'atome de carbone porteur du groupe éthényle a la configuration sinister (S) ou rectus (R), partiellement ou totalement salifiés.

Les composés de formule (I) pour lesquels $R_1$ est un groupe alkyle ou phénylalkyle peuvent aussi être préparés par action sur les composés correspondants de formule (I) pour lesquels $R_1$ est l'atome d'hydrogène d'un agent alkylant tel qu'un halogénure de formule $R''_1Hal$, un sulfate de formule $(R''_1)_2SO_4$, un arylsulfonate de formule $ArSO_3R''_1$ ou un alkylsulfonate de formule $R''SO_3R''_1$, formules dans lesquelles $R''_1$ représente un groupe alkyle ayant 1 à 4 atomes de carbone ou un groupe phénylakyle dont la partie alkyle comporte 1 à 3 atomes de carbone, Ar représente un groupe aryle et R'' représente un groupe alkyle. La réaction peut être schématisée comme suit dans le cas où l'agent alkylant est un halogénure :

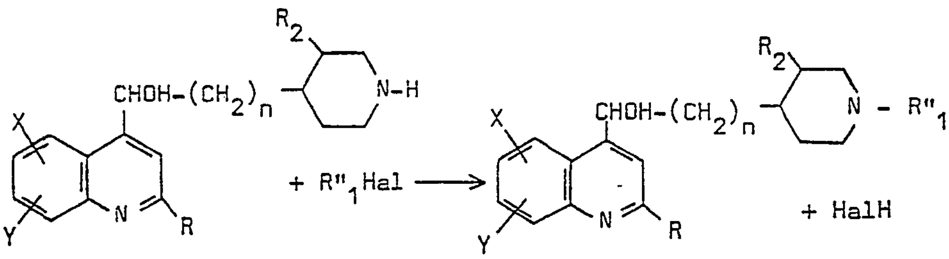


La réaction de l'agent alkylant avec les composés de formule (I) pour lesquels $R_1 = H$ est effectuée selon des procédés connus en soi. On opère avantageusement en présence d'une base organique ou minérale (par exemple le carbonate de sodium ou de potassium), au sein d'un solvant inerte, par exemple le diméthylformamide.

Une variante intéressante pour la préparation des composés de formule (I) pour lesquels $R_1 = CH_3$ consiste à faire agir sur les composés correspondants de formule (I) pour lesquels $R_1 = H$ le formaldéhyde en présence d'un réducteur (cf. « Complex hydrides and related reducing agents in organic synthesis » déjà cité). Comme agent réducteur on utilise avantageusement un borohydrure tel que le borohydrure de sodium ou de potassium ou la cyanoborohydrure de sodium, au sein d'un solvant inerte, par exemple un alcool ou un mélange eau + alcool, à une température comprise entre la température ambiante et le point d'ébullition du solvant.

Lorsque $R_2$ représente le groupe éthényle et si on opère à une température suffisante (> 50 °C), on observe simultanément, lors de l'action du formaldéhyde en présence du réducteur, l'épimérisation du groupe éthényle, si bien qu'en une seule réaction on peut préparer, à partir d'un seul précurseur, deux diastéréoisomères différant l'un de l'autre par la configuration de l'atome de carbone portant le groupe éthényle. La réaction peut être schématisée comme suit :

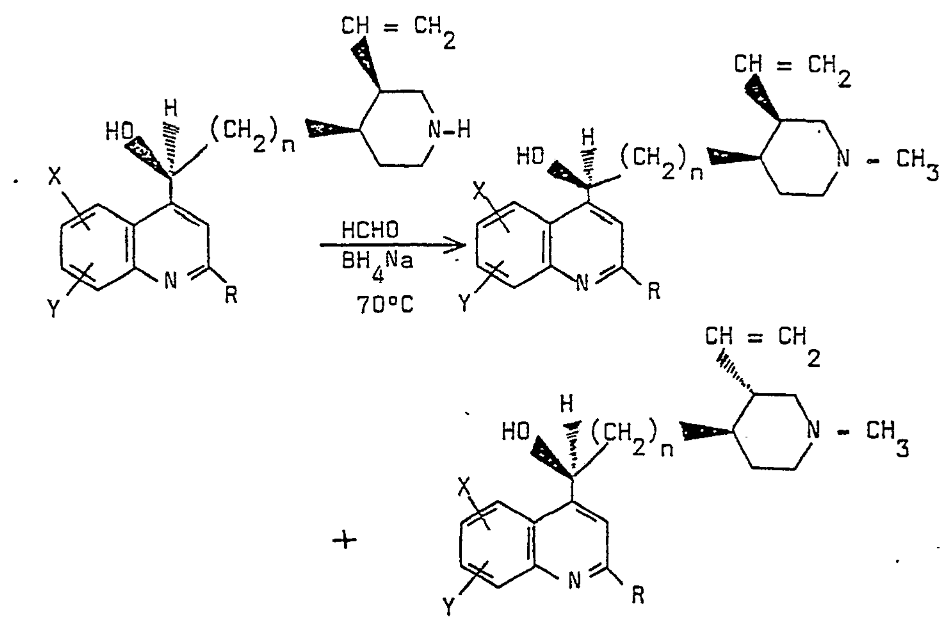

Chaque diastéréoisomère peut être isolé à l'état pur à partir du mélange par les techniques indiquées plus haut.

Les composés de formule (I) sous forme de base libre peuvent être éventuellement transformés en sels d'addition avec un acide minéral ou organique par action d'un tel acide au sein d'un solvant approprié.

Certaines des cétones de formule (II) sont connues. C'est le cas en particulier de la quinicine et de la cinchonicine, qui sont obtenues par réarrangement en milieu acide des alcaloïdes majeurs du quinquina, c'est-à-dire de la quinine ou quinidine et de la cinchonine ou cinchonidine (cf. S. W. PELLETIER, Chemistry of the Alkaloïds, p. 313, Reinhold, 1969), et qui répondent à la formule :

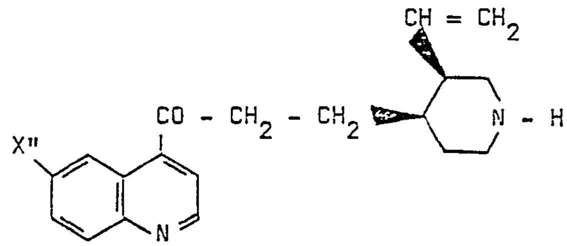

(II bis)

dans laquelle X" est un atome d'hydrogène ou un groupe méthoxy.

D'une façon générale, les cétones de formule (II) pour lesquelles $R_1$ représente l'atome d'hydrogène peuvent être préparées par condensation d'un ester d'acide quinoléine-carboxylique-4 de formule (III) avec un ester d'acide (pipéridinyl-4) alkylcarboxylique de formule (IV), puis hydrolyse et décarboxylation du composé de formule (V) ainsi obtenu, selon le schéma réactionnel suivant :

a)

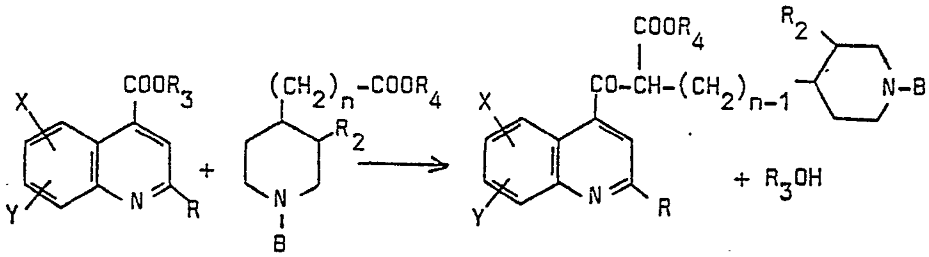


(III) (IV) (V)

b)

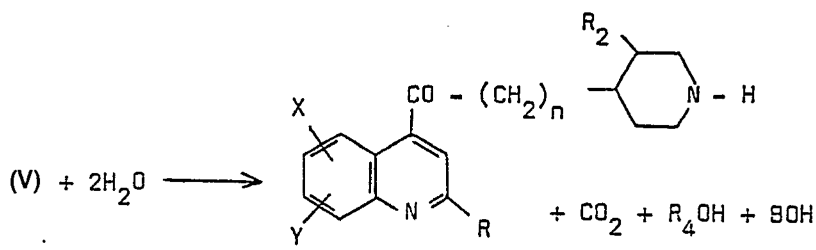


Dans les formules (III), (IV) et (V) ci-dessus, X, Y, R, $R_2$ et n ont la même signification que dans la formule (I), $R_3$ et $R_4$ représentent des groupes alkyle de bas poids moléculaire, par exemple méthyle ou éthyle, et B représente un groupe protecteur de la fonction amine, stable en milieu alcalin anhydre et susceptible d'être éliminé en milieu acide, tels que ceux qui sont décrits par R.A. BOISSONNAS, Advances in Organic Chemistry *3*, p. 159, Interscience (1963). On utilise avantageusement le groupe benzoyle ($—B = —CO—C_6H_5$) ou le groupe benzyloxycarbonyle ($—B = —CO—O—CH_2—C_6H_5$).

Pour réaliser la réaction de condensation a) on fait appel à des procédés connus en soi (cf. « The acetoacetic acid ester condensation », C.R. HAUSER et coll., Organic Reactions, vol. 1, p. 266, WILEY and Sons, 1942). On opère avantageusement en présence d'une base telle qu'un alcoolate (par exemple le tertiobutylate de potassium) ou un hydrure métallique (par exemple l'hydrure de sodium ou de potassium), au sein d'un solvant inerte tel qu'un hydrocarbure ou un autre solvant aprotique (par exemple le tétrahydrofuranne), à une température comprise entre 0 °C et la température d'ébullition du solvant utilisé.

La réaction d'hydrolyse b) est conduite selon des procédés connus en soi (cf. « Cleavage of β-keto-esters », R.B. WAGNER et H.D. ZOOK, Synthetic Organic Chemistry, p. 327, WILEY and Sons, 1953). La méthode la plus courante consiste à chauffer à l'ébullition le produit de formule (V) dans une solution aqueuse d'un acide tel que l'acide chlorhydrique ou l'acide sulfurique.

Les cétones de formule (II) pour lesquelles $R_1$ ne représente pas l'atome d'hydrogène peuvent être préparées par action d'un agent alkylant sur les cétones de formule (II) pour lesquelles $R_1 = H$. Cette alkylation est effectuée dans les conditions indiquées plus haut pour l'alkylation des composés de formule (I) pour lesquels $R_1 = H$.

Les exemples suivants illustrent la préparation des composés de formule (I), substances actives des médicaments selon l'invention. Dans ces exemples, la configuration absolue de l'atome de carbone porteur du groupe OH des composés synthétisés a été déterminée par la méthode de J.A. DALE et H.S. MOSHER, J. Amer. Chem. Soc., 1973, *95*, 512.

## Exemple 1

(Phényl-2 quinolyl-4)-1 (pipéridyl-4)-2 éthanol (racémique).

A 16 g de dichlorhydrate de (phényl-2 quinolyl-4)-1 (pipéridyl-4)-2 éthanone dans 500 ml de méthanol on ajoute en 20 minutes, à la température ambiante, 6 g de borohydrure de sodium. Après 2 heures de réaction à la température ambiante, on ajoute 350 ml d'eau et élimine le méthanol par distillation sous pression réduite. On extrait la suspension aqueuse résiduelle avec de l'oxyde diéthylique, lave la phase organique à l'eau, la sèche sur du sulfate de magnésium et l'évapore sous pression réduite. Le résidu (13 g) est cristallisé dans de l'éther de pétrole. On obtient ainsi 9 g de (phényl-2 quinolyl-4)-1 (pipéridyl-4)-2 éthanol (racémique), qui fond à 147 °C.

Le produit de départ peut être préparé comme suit :

A une suspension de 27,5 g de t-butylate de potassium dans 215 ml de tétrahydrofuranne sec, placée sous atmosphère d'azote et refroidie à 0 °C, on ajoute rapidement une solution de 21,2 g de phényl-2 quinoléinecarboxylate-4 de méthyle dans 50 ml de tétrahydrofuranne sec. La température étant maintenue inférieure à + 10 °C, on introduit lentement en 2 heures une solution de 22,1 g de (benzoyl-1 pipéridyl-4) acétate d'éthyle dans 80 ml de tétrahydrofuranne sec. Le mélange réactionnel est ensuite agité pendant 20 heures à température ambiante, puis on amène à sec par évaporation du solvant. Le résidu est chauffé au reflux pendant 18 heures dans 650 ml d'une solution aqueuse 5N d'acide chlorhydrique.

Après refroidissement, la solution obtenue est filtrée et le filtrat extrait par 2 fois 250 ml d'éther. La solution aqueuse résiduelle est concentrée sous pression réduite. Le résidu obtenu est extrait par 500 ml de méthanol chaud et la solution d'extraction est filtrée. Le filtrat, après évaporation du méthanol, fournit 13,8 g de dichlorhydrate de (phényl-2 quinolyl-4)-1 (pipéridyl-4)-2 éthanone fondant à 259 °C.

## Exemple 2

(Quinolyl-4)-1 (pipéridyl-4)-3 propanol-1 (racémique).

A 13 g de (quinolyl-4)-1 (pipéridyl-4)-3 propanone-1 dans 200 ml de méthanol on ajoute, en 20 minutes, à la température ambiante, 2 g de borohydrure de sodium. Après 2 heures de réaction à la température ambiante, on acidifie le milieu réactionnel par addition d'une solution aqueuse d'acide chlorhydrique, élimine le méthanol par distillation sous pression réduite et lave la phase aqueuse avec de l'acétate d'éthyle. La phase aqueuse est alcalinisée par addition d'une solution aqueuse d'hydroxyde de sodium, puis est extraite avec du chloroforme. La phase chloroformique est lavée à l'eau, séchée sur sulfate de magnésium et évaporée sous pression réduite. On obtient ainsi 13 g de produit brut, qui sont fixés sur une colonne de gel de silice. On élue ensuite avec un mélange de 90 parties en volume de chloroforme et 10 parties en volume de diéthylamine. On isole ainsi 7 g du produit recherché à l'état de base, que l'on transforme en dichlorhydrate par action de l'acide chlorhydrique au sein de l'éthanol. On obtient ainsi 3,2 g de dichlorhydrate de (quinolyl-4)-1 (pipéridyl-4)-3 propanol-1 (racémique), qui fond à 195 °C.

La cétone de départ peut être préparée comme indiqué par P. RABE, Ber., *55*, 532 (1922).

## Exemple 3

(Méthoxy-6 quinolyl-4)-1 (pipéridyl-4)-3 propanol-1 (racémique).

On opère comme à l'exemple 2, sauf que l'on part de 6,6 g de (méthoxy-6 quinolyl-4)-1 (pipéridyl-4)-3 propanone-1 et de 1 g de borohydrure de sodium dans 100 ml de méthanol et que le produit recherché à l'état de base est transformé en son sesquifumarate. On obtient ainsi 5 g de sesquifumarate de (méthoxy-6 quinolyl-4)-1 (pipéridyl-4)-3 propanol-1 (racémique), qui fond à 154 °C.

La cétone de départ est préparée comme indiqué par M. KLEIMAN J. Org. Chem., 1945, *10*, 562.

## Exemple 4

(Phényl-2 quinolyl-4)-1 (pipéridyl-4)-3 propanol-1 (racémique).

On opère comme à l'exemple 1, sauf que l'on part de 14 g de (phényl-2 quinolyl-4)-1 (pipéridyl-4)-3 propanone-1 et 2,3 g de borohydrure de sodium dans 300 ml de méthanol. Après recristallisation du produit brut dans l'isopropanol, on obtient 7 g de (phényl-2 quinolyl-4)-1 (pipéridyl-4)-3 propanol-1 (racémique), qui fond à 162 °C.

La cétone de départ peut être préparée comme indiqué dans le brevet belge n° 807 491.

Exemple 5

Mélange d'[éthényl-3(R) pipéridyl-4(R)]-3 (méthoxy-6 quinolyl-4)-1 propanol-1(R) et d'[éthényl-3(R) pipéridyl-4(R)]-3 (méthoxy-6 quinolyl-4)-1 propanol-1(S).

A 194 g de monochlorhydrate d'[éthényl-3(R) pipéridyl-4(R)]-3 (méthoxy-6 quinolyl-4)-1 propanone-1 et 32 g de méthylate de sodium dans 2 200 ml de méthanol on ajoute 26,6 g de borohydrure de sodium. Après 2 heures d'agitation à la température ambiante, on filtre et évapore le méthanol sous pression réduite. On reprend le résidu avec 1 litre de chlorure de méthylène et 500 ml d'eau, extrait, sépare les phases et extrait à nouveau la phase aqueuse avec 500 ml de chlorure de méthylène.

Les phases organiques rassemblées sont lavées avec 3 fois 200 ml d'eau, séchées sur sulfate de magnésium et évaporées sous pression réduite. L'huile résiduelle est dissoute dans 500 ml d'éthanol absolu et le milieu est amené à pH 3 par addition d'une solution 10N d'acide chlorhydrique dans l'éthanol. Les cristaux formés sont filtrés, lavés et séchés. On obtient ainsi 144 g d'un mélange d'[éthényl-3(R) pipéridyl-4(R)]-3 (méthoxy-6 quinolyl-4)-1 propanol-1(R) et d'[éthényl-3(R) pipéridyl-4(R)]-3 (méthoxy-6 quinolyl-4)-1 propanol-1(S) sous forme de dichlorhydrates, mélange qui fond à 223-225 °C. L'analyse par chromatographie liquide haute pression de ce mélange montre qu'il s'agit d'un mélange 50/50 des deux diastéréoisomères.

La cétone de départ (chlorhydrate de quinicine) peut être préparée comme indiqué par A. QUEVAUVILLER et Coll. Ann. Pharm. Franc. *24*. 39 (1966).

Exemple 6

[Ethényl-3(R) pipéridyl-4(R)]-3 méthoxy-6 quinolyl-4)-1 propanol-1(S).

Le produit obtenu à l'exemple 5 est recristallisé trois fois dans l'éthanol à 95 %. On obtient ainsi 18 g d'[éthényl-3(R) pipéridyl-4(R)]-3 (méthoxy-6 quinolyl-4)-1 propanol-1(S) sous forme de dichlorhydrate fondant à 245-248 °C.

Pouvoir rotatoire du produit obtenu (mesuré sur une solution aqueuse à 2 %) :

$$\alpha^{21}_D = -122°8$$

Exemple 7

[Ethényl-3(R) pipéridyl-4(R)]-3 (méthoxy-6 quinolyl-4)-1 propanol-1(R).

Le filtrat provenant de la première recristallisation effectuée à l'exemple 6 est évaporé. Le résidu est recristallisé une fois dans l'isopropanol, puis trois fois dans un mélange éthanol absolu-isopropanol 1/1. On obtient ainsi 8 g d'[éthényl-3(R) pipéridyl-4(R)]-3 (méthoxy-6 quinolyl-4)-1 propanol-1(R) sous forme de dichlorhydrate fondant à 220-222 °C.

Pouvoir rotatoire du produit obtenu (mesuré sur une solution aqueuse à 2 %) :

$$\alpha^{21}_D = +197°9$$

Exemple 8

[Ethyl-3(R) pipéridyl-4(R)]-3 (méthoxy-6 quinolyl-4)-1 propanol-1(S) et [éthyl-3(R) pipéridyl-4(R)]-3 (méthoxy-6 quinolyl-4)-1 propanol-1(R).

On hydrogène à la température ambiante, sous une pression d'hydrogène égale à la pression atmosphérique et en présence de 23 g de palladium sous forme de charbon palladié à 10 % de palladium, 180 g de monochlorhydrate d'[éthényl-3(R) pipéridyl-4(R)]-3 (méthoxy-6 quinolyl-4)-1 propanone-1 dans 2 250 ml d'éthanol absolu et 250 ml d'une solution aqueuse 2N d'acide chlorhydrique. Lorsque l'absorption d'hydrogène est terminée, on filtre le mélange réactionnel et l'évapore sous pression réduite. L'huile résiduelle est reprise dans 500 ml d'éthanol chaud, et on ajoute 500 ml d'acétone pour déclencher la cristallisation. Les cristaux formés sont filtrés, lavés et séchés. On obtient ainsi 143 g d'un produit brut qui est un mélange en parties égales des dichlorhydrates d'[éthyl-3(R) pipéridyl-4(R)]-3 (méthoxy-6 quinolyl-4)-1 propanol-1(S) et d'[éthyl-3(R) pipéridyl-4(R)]-3 (méthoxy-6 quinolyl-4)-1 propanol-1(R).

Le produit brut ci-dessus est recristallisé trois fois dans l'éthanol à 95 % et les cristaux de

dichlorhydrate ainsi isolés sont transformés en la base correspondante par action de l'hydroxyde de sodium. Cette base est fixée sur une colonne de gel de silice et on élue ensuite avec un mélange contenant 1 partie en volume de chloroforme, 0,1 partie en volume de méthanol et 0,025 partie en volume de diéthylamine. On obtient ainsi 11,3 g de produit à l'état de base, que l'on retransforme en dichlorhydrate par action de l'acide chlorhydrique au sein de l'éthanol absolu. Après une recristallisation de ce dichlorhydrate dans l'éthanol absolu, on isole 7 g de dichlorhydrate d'[éthyl-3(R) pipéridyl-4(R)]-3 (méthoxy-6 quinolyl-4)-1 propanol-1(S), qui fond à 228-230 °C et dont le pouvoir rotatoire, mesuré sur une solution aqueuse à 2 %, est :

$$\alpha^{21}_D = -144°2$$

Le filtrat (environ 1 300 ml) provenant de la première recristallisation du produit brut dans l'éthanol à 95 % est concentré jusqu'à ce que son volume soit réduit de moitié. On filtre, puis concentre à nouveau le filtrat jusqu'à réduction de moitié de son volume. On filtre encore, et évapore à sec, sous pression réduite, le filtrat résiduel. On obtient ainsi 29 g de produit à l'état de dichlorhydrate, qui est transformé en la base correspondante par action de l'hydroxyde de sodium. Cette base est fixée sur une colonne de gel de silice et on élue ensuite avec un mélange chloroforme-méthanol-diéthylamine 1/0,1/0,025. Le produit ainsi isolé à l'état de base est transformé en dichlorhydrate par action de HCl au sein de l'éthanol absolu, puis recristallisé dans le n-propanol. On obtient ainsi 5 g de dichlorhydrate d'[éthyl-3(R) pipéridyl-4(R)]-3 (méthoxy-6 quinolyl-4)-1 propanol-1(R), qui fond à 210-215 °C et dont le pouvoir rotatoire, mesuré sur une solution aqueuse à 2 %, est :

$$\alpha^{21}_D = +157°4$$

## Exemple 9

[Ethényl-3(R) méthyl-1 pipéridyl-4(R)]-3 (méthoxy-6 quinolyl-4)-1 propanol-1(S) et [éthényl-3(S) méthyl-1 pipéridyl-4(R)]-3 (méthoxy-6 quinolyl-4)-1 propanol-1(S).

On traite 2 heures, à 70 °C, 8 g d'éthényl-3(R) pipéridyl-4(R)]-3 (méthoxy-6 quinolyl-4)-1 propanol-1(S) (produit de l'exemple 6) par 24 ml d'une solution aqueuse à 37 % de formaldéhyde et 3,4 g de borohydrure de sodium dans 100 ml de méthanol. On évapore le solvant sous pression réduite, reprend le résidu dans de l'eau, alcalinise la phase aqueuse et l'extrait au moyen de chloroforme. On lave la phase organique à l'eau, la sèche sur du sulfate de magnésium et l'évapore sous pression réduite.

Le résidu est soumis à une chromatographie en phase liquide sous pression (support : silice ; éluant : mélange de 9 parties en volume de toluène et 1 partie en volume de diéthylamine). Les produits recherchés, qui se trouvent à l'état de base dans les fractions séparées, sont transformés en chlorhydrates par action de HCl au sein de l'éthanol. On obtient ainsi d'une part 2,5 g d'[éthényl-3(R) méthyl-1 pipéridyl-4(R)]-3 (méthoxy-6 quinolyl-4)-1 propanol-1(S) sous forme de monochlorhydrate, qui fond à 214 °C et dont le pouvoir rotatoire (mesuré sur une solution aqueuse à 2 %) est $\alpha_D^{23} = -61°9$, et d'autre part 2,7 g d'[éthényl-3(S) méthyl-1 pipéridyl-4(R)]-3 (méthoxy-6 quinolyl-4)-1 propanol-1(S) sous forme de dichlorhydrate, qui fond à 175 °C et dont le pouvoir rotatoire (mesuré sur une solution aqueuse à 2 % est $\alpha_D^{22} = -172°8$.

## Exemple 10

[Ethényl-3(R) méthyl-1 pipéridyl-4(R)]-3 (méthoxy-6 quinolyl-4)-1 propanol-1(R).

On opère comme à l'exemple 9, en partant de 1,15 g d'[éthényl-3(R) pipéridyl-4(R)]-3 (méthoxy-6 quinolyl-4)-1 propanol-1(R) (produit de l'exemple 7), 3,5 ml de solution aqueuse à 37 % de formaldéhyde et 0,5 g de borohydrure de sodium dans 15 ml de méthanol. Après séparation par chromatographie en phase liquide sous pression, on isole 0,5 g d'[éthényl-3(R) méthyl-1 pipéridyl-4(R)]-3 (méthoxy-6 quinolyl-4)-1 propanol-1(R) sous forme de monochlorhydrate, qui fond à 160-165 °C et dont le pouvoir rotatoire (mesuré sur une solution aqueuse à 2 %) est $\alpha_D^{23} = +199°0$.

## Exemple 11

Mélange des deux isomères [Ethényl-3(R) pipéridyl-4(R)]-3 (méthoxy-6 quinolyl-4)-1 propanol-1(R) et [éthényl-3(R) pipéridyl-4(R)]-3 (méthoxy-6 quinolyl-4)-1 propanol-1(S).

On agite 20 heures, à la température ambiante, 0,095 g de borohydrure de sodium et 0,29 g de L-proline dans 5 ml de tétrahydrofuranne sec. On ajoute ensuite 0,9 g de monochlorhydrate d'[éthényl-3(R) pipéridyl-4(R)]-3 (méthoxy-6 quinolyl-4)-1 propanone-1 et poursuit l'agitation du mélange réactionnel pendant 4 jours. On évapore le solvant sous pression réduite, reprend le résidu dans l'eau, alcalinise la phase aqueuse et extrait la phase aqueuse avec du chlorure de méthylène. On sèche la phase organique

sur du sulfate de magnésium et l'évapore sous pression réduite. On obtient 0,8 g d'une huile qui, soumise à l'action de HCl au sein de l'éthanol, fournit 0,4 g d'un mélange des dichlorhydrates d'[éthényl-3(R) pipéridyl-4(R)]-3 (méthoxy-6 quinolyl-4)-1 propanol-1(R) et d'[éthényl-3(R) pipéridyl-4(R)]-3 (méthoxy-6 quinolyl-4)-1 propanol-1(S), mélange qui fond à 223-225 °C. Ce mélange contient 90 % d'isomère 3R, 4R, 1S et 10 % d'isomère 3R, 4R, 1R, ainsi que le montre l'analyse par chromatographie en phase liquide haute pression du mélange des bases correspondant.

Exemple 12

[(Diméthyl-1,1 éthyl)-2 quinolyl-4]-1 (pipéridyl-4)-3 propanol-1 (racémique).

On opère comme à l'exemple 5, en partant de 14 g de dichlorhydrate de [(diméthyl-1,1 éthyl)-2 quinolyl-4]-1 (pipéridyl-4)-3 propanone-1, 4,15 g de méthylate de sodium et 1,8 g de borohydrure de sodium dans 150 ml de méthanol. On obtient finalement 4,1 g de [(diméthyl-1,1 éthyl)-2 quinolyl-4]-1 (pipéridyl-4)-3 propanol-1 (racémique), sous forme de dichlorhydrate fondant à 219 °C.

La cétone de départ peut être préparée de la façon suivante :

A une solution de 48 g de (diméthyl-1,1 éthyl)-2 quinoléine-carboxylate-4 d'éthyle dans 800 ml de tétrahydrofuranne anhydre, placée sous atmosphère d'azote, on ajoute 29,6 g d'une suspension à 80 % d'hydrure de sodium dans l'huile. On porte à l'ébullition et ajoute, en 2 heures, une solution de 47 g de (benzoyl-1 pipéridyl-4)-3 propionate d'éthyle dans 100 ml de tétrahydrofuranne anhydre. On maintient ensuite l'ébullition pendant 2 heures. Après refroidissement, on ajoute 100 ml d'éthanol, évapore à sec, reprend le résidu à l'eau et amène la solution aqueuse à pH 6 par addition d'acide acétique. On extrait l'insoluble avec 3 fois 300 ml d'acétate d'éthyle, lave la phase organique à l'eau, la sèche sur du sulfate de magnésium et l'évapore sous pression réduite. Le résidu (36 g) est chauffé au reflux, pendant 19 heures, dans 500 ml d'une solution aqueuse 5N d'acide chlorhydrique. On alcalinise la solution aqueuse avec une lessive d'hydroxyde de sodium, extrait l'insoluble avec 3 fois 300 ml de chloroforme, lave la phase organique à l'eau, la sèche sur du sulfate de magnésium et l'évapore sous pression réduite. On obtient ainsi 35 g de produit brut qui, soumis à l'action de l'acide chlorhydrique au sein de l'éthanol, fournissent 28 g de [(diméthyl-1,1 éthyl)-2 quinolyl-4]-1 (pipéridyl-4)-3 propanone-1, sous forme de dichlorhydrate fondant à 200 °C.

Le (diméthyl-1,1 éthyl)-2 quinoléinecarboxylate-4 d'éthyle peut être préparé comme indiqué par J.P. SCHAEFER et Coll. (J. Heterocycl. Chemistry, 1970, 607).

Exemple 13

[(Diméthyl-1,1 éthyl)-2 quinolyl-4]-1 [(phényl-2 éthyl)-1 pipéridyl-4]-3 propanol-1 (racémique).

On opère comme à l'exemple 5, en partant de 10 g de dichlorhydrate de [(diméthyl-1,1 éthyl)-2 quinolyl-4]-1 [(phényl-2 éthyl)-1 pipéridyl-4]-3 propanone-1, 2,2 g de méthylate de sodium et 0,8 g de borohydrure de sodium dans 250 ml de méthanol. On obtient 8,4 g de dichlorhydrate de [(diméthyl-1,1 éthyl)-2 quinolyl-4]-1 [(phényl-2 éthyl)-1 pipéridyl-4]-3 propanol-1 (racémique), qui fond à 190 °C.

La cétone de départ peut être préparée de la manière suivante :

On chauffe 7 heures à 70 °C un mélange de 14 g de dichlorhydrate de [(diméthyl-1,1 éthyl)-2 quinolyl-4]-1 (pipéridyl-4)-3 propanone-1, 9 g de bromure de (phényl-2) éthyle et 21,3 g de carbonate de potassium dans 140 ml de diméthylformamide. On élimine ensuite le diméthylformamide par distillation sous pression réduite et reprend le résidu par 400 ml et 200 ml de toluène. On sépare la phase organique, la lave avec 200 ml d'eau, la sèche et l'évapore sous pression réduite. Le produit brut ainsi obtenu fournit, par action de l'acide chlorhydrique au sein de l'éthanol, 13,7 g de dichlorhydrate de [(diméthyl-1,1 éthyl)-2 quinolyl-4]-1 [(phényl-2 éthyl)-1 pipéridyl-4]-3 propanone-1, qui fond à 130 °C.

Exemple 14

(Méthyl-1 pipéridyl-4)-2 (phényl-2 quinolyl-4)-1 éthanol (racémique).

On agite 2 heures, à la température ambiante, un mélange de 2,55 g de (phényl-2 quinolyl-4)-1 (pipéridyl-4)-2 éthanol (racémique), 1,1 g d'iodure de méthyle et 0,6 g de carbonate de potassium dans 20 ml de diméthylformamide. On ajoute ensuite 20 ml d'eau et 30 ml de toluène. On sépare la phase organique, la lave à l'eau, la sèche sur du sulfate de magnésium et l'évapore sous pression réduite. On obtient 1 g de produit brut qui est fixé sur une colonne de gel de silice. On élue avec un mélange chloroforme-diéthylamine 9/1 et isole ainsi 0,57 g de (méthyl-1 pipéridyl-4)-2 (phényl-2 quinolyl-4)-1 éthanol (racémique), qui fond à 208 °C.

Exemple 15

[Ethényl-3(S) pipéridyl-4(R)]-3 (méthoxy-6 quinolyl-4)-1 propanol-1(S).

On chauffe à 120 °C pendant 24 heures, dans un autoclave, un mélange de 6 g de dichlorhydrate d'[éthényl-3(R) pipéridyl-4(R)]-3 (méthoxy-6 quinolyl-4)-1 propanol-1(S), 15 ml de solution aqueuse normale d'hydroxyde de sodium, 1,2 ml d'une solution aqueuse à 0,4 % de formaldéhyde et 60 ml d'eau. Après refroidissement, on alcalinise le milieu réactionnel par addition de lessive d'hydroxyde de sodium et extrait avec du chlorure de méthylène. La phase organique est lavée à l'eau, séchée sur sulfate de magnésium et évaporée sous pression réduite. Le résidu huileux obtenu est soumis à une chromatographie en phase liquide sous pression (support : gel de silice ; éluant : mélange toluène-méthanol-diéthylamine 100/7,5/3,75). Les fractions séparées contenant le produit recherché sont évaporées. On récupère ainsi 2,69 d'une huile qui, par action de HCl au sein de l'éthanol, est transformée en dichlorhydrate. Après trois recristallisations de ce dernier produit dans l'éthanol, on obtient 0,7 g d'[éthényl-3(S) pipéridyl-4(R)]-3 (méthoxy-6 quinolyl-4)-1 propanol-1(S) sous forme de dichlorhydrate fondant à 204 °C.

Exemple 16

(Cyclohexyl-2 quinolyl-4)-1 (pipéridyl-4)-3 propanol-1 (racémique).

On opère comme à l'exemple 5, en partant de 5,1 g de monochlorhydrate de (cyclohexyl-2 quinolyl-4)-1 (pipéridyl-4)-3 propanone-1, 1,3 ml d'une solution aqueuse 10N d'hydroxyde de sodium (à la place de méthylate de sodium) et 0,53 g de borohydrure de sodium dans 100 ml d'éthanol. On obtient finalement 1,3 g de (cyclohexyl-2 quinolyl-4)-1 (pipéridyl-4)-3 propanol-1 (racémique) sous forme de sulfate neutre fondant à 260 °C.

La cétone de départ peut être préparée comme suit :

A 15 g d'une suspension à 80 % d'hydrure de sodium dans l'huile, placée sous atmosphère d'azote, on ajoute 350 ml de tétrahydrofuranne anhydre et 27 g de cyclohexyl-2 quinoléinecarboxylate-4 d'éthyle. On porte à l'ébullition, ajoute 24 g de (benzoyl-1 pipéridyl-4)-3 propionate d'éthyle en solution dans 100 ml de tétrahydrofuranne anhydre et 12 g d'éthylate de potassium dans 45 ml de diméthylformamide. On maintient ensuite l'ébullition pendant une heure, puis refroidit et ajoute 50 ml d'éthanol. On élimine les solvants par distillation sous pression réduite, ajoute de l'eau, amène le pH de la solution à 6 par addition d'acide acétique et extrait avec de l'acétate d'éthyle. On lave la phase organique à l'eau, la sèche sur du sulfate de magnésium anhydre et l'évapore sous pression réduite jusqu'à siccité. On reprend le résidu dans 500 ml d'une solution aqueuse concentrée d'acide chlorhydrique et chauffe le mélange 23 heures à l'ébullition. Après refroidissement, on alcalinise la solution par addition d'une solution aqueuse 10N d'hydroxyde de sodium et extrait l'insoluble avec du chlorure de méthylène. On lave la phase organique à l'eau, la sèche sur du sulfate de magnésium anhydre et l'évapore sous pression réduite jusqu'à siccité. Le résidu est fixé sur une colonne de gel de silice et on élue avec un mélange chloroforme-diéthylamine 95/5. On obtient ainsi 14 g du produit recherché à l'état de base libre, qui est transformée ensuite en monochlorhydrate par action de l'acide chlorhydrique au sein de l'éthanol. Après recristallisation dudit monochlorhydrate dans l'éthanol, on isole 6,5 g de monochlorhydrate de (cyclohexyl-2 quinolyl-4)-1 (pipéridyl-4)-3 propanone-1 qui fond à 190-191 °C.

Le cyclohexyl-2 quinoléinecarboxylate-4 d'éthyle peut être préparé selon la méthode de J.F. MEAD et Coll., J. Am. Chem. Soc., 1946, 68, 2708.

Propriétés pharmacologiques des composés de formule (I) :

L'activité antiarythmique des composés de formule (I) a été démontrée à l'aide de deux tests : le test à l'aconitine chez le rat et le test au chloroforme chez la souris.

Test à l'aconitine

Le principe de la technique repose sur le temps d'induction des arythmies ventriculaires provoquées par l'aconitine en perfusion lente chez le rat. Une substance antiarythmique retarde l'apparition des arythmies et ce délai est proportionnel à l'activité de la substance.

On utilise des groupes de 5 rats mâles. Une anesthésie individuelle est réalisée (uréthane 10 % : 1 g/kg/ip) pour permettre une cathétérisation de la veine du pénis. L'électrocardiogramme est enregistré. Au temps T = 0 la substance étudiée est injectée sous forme d'une solution aqueuse, à raison de 2,5 ml de solution par kg en 30 secondes. Au temps T = 60 secondes, soit 30 secondes après la fin de l'injection, l'aconitine est perfusée à raison de 20 µg par minute, jusqu'à l'apparition d'extra systoles supraventriculaires. Le temps de perfusion de l'aconitine est noté.

On exprime les résultats par une $DE_{50}$, dose de produit en mg/kg qui, par rapport aux animaux témoins, augmente de 50 % le temps de perfusion de l'aconitine.

Test au chloroforme

On utilise la technique de LAWSON (J. Pharm. Exp. Therap., *160*, 2231, 1968), qui consiste à rechercher une

protection éventuelle contre les fibrillations engendrées par une inhalation de chloroforme poursuivie jusqu'à l'arrêt respiratoire. Le produit à tester est administré par voie intrapéritonéale 20 minutes avant l'intoxication chloroformique et la protection éventuelle contre l'arythmie est mise en évidence par l'enregistrement de l'électrocardiogramme, effectué dès l'apparition de l'apnée. L'activité des produits est exprimée par une $DA_{50}$ (dose de produit en mg/kg qui protège 50 % des animaux).

Les résultats obtenus sont rassemblés dans le tableau ci-dessous, où figurent également les données toxicologiques.

| PRODUIT DE L'EXEMPLE | Toxicité aigüe chez la souris i.v. | Activité antiarythmique | |
|---|---|---|---|
| | | Test à l'aconitine chez le rat i.v. | Test au chloroforme chez la souris, i.p. |
| | $DL_{50}$ mg/kg | $DE_{50}$ mg/kg | $DA_{50}$ mg/kg |
| 4 | 21 | 1,22 | 2,5 |
| 5 | 21,5 | 0,44 | 3 |
| 6 | 20,3 | 0,43 | 3 |
| 7 | 33 | 0,45 | 3 |
| 8(isomère propanol-1(S)) | 17 | 0,58 | 2,5 |
| 8 (isomère propanol-1(R)) | 29 | 0,65 | 2,5 |
| 9 (isomère éthényl-3(R)) | 19 | 1 | 5 |
| 9 (isomère éthényl-3(S)) | 33 | 1,4 | > 5 |
| 12 | 26 | 0,8 | - |
| Sulfate de quinidine (produit de référence) | 60 | 7,5 | 18 |

On voit que les composés de formule (I) présentent de remarquables propriétés antiarythmiques et sont plus actifs que la quinidine.

Propriétés toxicologiques des composés de formule (I) :

Les toxicités aiguës des composés de formule (I) (voir résultats dans le tableau ci-dessus) ont été déterminées chez la souris mâle $CD_1$ (Charles RIVER), par la voie intraveineuse. Les $DL_{50}$ ont été calculées, après 3 jours d'observation, par la méthode cumulative de J.J. Reed et H. Muench (Amer. J. Hyg., *27*, 493, 1938).

Utilisation thérapeutique

Les médicaments selon l'invention qui contiennent un composé de formule (I) ou un mélange de

composés stéréoisomères répondant à la formule (I) ou un sel d'un tel composé ou mélange de composés stéréoisomères avec un acide pharmaceutiquement acceptable, associé à un véhicule pharmaceutiquement acceptable, peuvent être utilisés en thérapeutique humaine pour le traitement et/ou la prévention des troubles du rythme. Ils peuvent se présenter sous toutes les formes en usage dans le domaine des médicaments, telles que comprimés, capsules, gélules, suppositoires, solutions ingérables ou injectables, etc...

La posologie dépend des effets recherchés et de la voie d'administration utilisée. Par exemple, par voie orale, elle peut être comprise entre 50 à 800 mg de substance active par 24 heures, avec des doses unitaires allant de 10 à 100 mg de substance active.

**Revendications**

1. Médicaments contenant une substance active et un véhicule pharmaceutiquement acceptable caractérisés en ce que la substance active est un composé répondant à la formule :

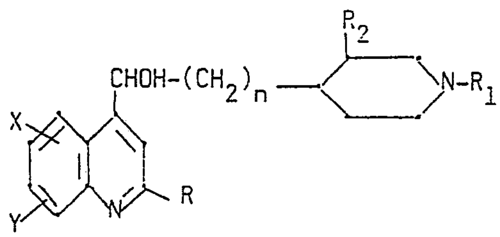

(I)

dans laquelle

a) soit n est égal à 1, X et Y qui sont identiques ou différents, sont fixés en position 5, 6, 7 ou 8 sur le cycle de la quinoléine et représentent chacun l'atome d'hydrogène ou un groupe alcoxy comportant 1 à 3 atomes de carbone, R représente l'atome d'hydrogène, un groupe cycloalkyle ayant 3 à 8 atomes de carbone, un groupe alkyle comportant 1 à 4 atomes de carbone, un groupe phényle ou un groupe phényle substitué par un groupe alcoxy ayant 1 à 4 atomes de carbone, $R_1$ représente l'atome d'hydrogène, un groupe alkyle comportant 1 à 4 atomes de carbone ou un groupe phénylalkyle dont la partie alkyle comporte 1 à 3 atomes de carbone, $R_2$ représente l'atome d'hydrogène, un groupe alkyle comportant 1 ou 2 atomes de carbone ou un groupe alcényle comportant 2 à 4 atomes de carbone.

b) soit n est égal à 2, $R_2$ représente l'atome d'hydrogène ou un groupe alcényle comportant 2 à 4 atomes de carbone, X, Y, R et $R_1$ ont les significations mentionnées ci-dessus en a),

c) soit n est égal à 2, $R_2$ représente un groupe alkyle comportant 1 ou 2 atomes de carbone, R représente un groupe cycloalkyle ayant 3 à 8 atomes de carbone, un groupe phényle ou un groupe phényle substitué par un groupe alcoxy ayant 1 à 4 atomes de carbone, X, Y et $R_1$ ont les significations mentionnées ci-dessus en a),

d) soit n est égal à 2, $R_2$ représente un groupe alkyle comportant 1 ou 2 atomes de carbone, R représente l'atome d'hydrogène, $R_1$ représente l'atome d'hydrogène ou un groupe phénylalkyle dont la partie alkyle comporte 1 à 3 atomes de carbone, X et Y ont les significations mentionnées ci-dessus en a),

e) soit n est égal à 2, $R_2$ représente un groupe alkyle comportant 1 ou 2 atomes de carbone, R représente un groupe alkyle comportant 1 à 4 atomes de carbone, $R_1$ représente un groupe phénylalkyle dont la partie alkyle comporte 1 à 3 atomes de carbone, X et Y ont les significations mentionnées ci-dessus en a),

ou un mélange de composés stéréoisomères répondant à la formule (I) ou un sel d'un tel composé ou mélange de composés stéréoisomères avec un acide pharmaceutiquement acceptable,

2. Médicaments selon la revendication 1, caractérisés en ce que, dans la formule (I), Y est un atome d'hydrogène, X est un atome d'hydrogène ou un groupe méthoxy, R est un atome d'hydrogène ou un groupe phényle ou tertiobutyle, $R_1$ est un atome d'hydrogène et $R_2$ est un atome d'hydrogène ou un groupe éthyle ou éthényle.

3. Médicaments selon la revendication 1, caractérisés en ce que dans la formule (I), n est égal à 1.

4. Médicaments selon la revendication 1, caractérisés en ce que, dans la formule (I), R est un groupe cycloalkyle ayant 3 à 8 atomes de carbone, un groupe alkyle ayant 1 à 4 atomes de carbone, un groupe phényle ou un groupe phényle substitué par un groupe alcoxy ayant 1 à 4 atomes de carbone.

5. Médicaments selon la revendication 1, caractérisés en ce que dans la formule (I), $R_2$ est un atome d'hydrogène.

6. Médicaments selon la revendication 2, caractérisés en ce que la substance active est un mélange d'[éthényl-3(R) pipéridyl-4(R)]-3 (méthoxy-6 quinolyl-4)-1 propanol-1(R) et d'[éthényl-3(R) pipéridyl-4 (R)]-3 (métoxy-6 quinolyl-4)-1 propanol-1(S) ou un sel d'un tel mélange avec un acide pharmaceutiquement acceptable.

7. Médicaments selon la revendication 2, caractérisés en ce que la substance active est l'[éthényl-3(R) pipéridyl-4(R)]-3 (méthoxy-6 quinolyl-4)-1 propanol-1(S) ou un sel de ce composé avec un acide pharmaceutiquement acceptable.

8. Médicaments selon la revendication 2, caractérisés en ce que la substance active est l'[éthényl-3(R) pipéridyl-4(R)]-3 (méthoxy-6 quinolyl-4)-1 propanol-1(R) ou un sel de ce composé avec un acide pharmaceutiquement acceptable.

9. Médicaments selon la revendication 2, caractérisés en ce que la substance active est le [(diméthyl-1,1 éthyl)-2 quinolyl-4]-1 (pipéridyl-4)-3 propanol-1 (racémique) ou un sel de ce produit avec un acide pharmaceutiquement acceptable.

10. Médicaments selon la revendication 2, caractérisés en ce que la substance active est l'[éthyl-3(R) pipéridyl-4(R)]-3 (méthoxy-6 quinolyl-4)-1 propanol-1(S) ou un sel de ce composé avec un acide pharmaceutiquement acceptable.

11. Médicaments selon la revendication 2, caractérisés en ce que la substance active est l'[éthyl-3(R) pipéridyl-4(R)]-3 (méthoxy-6 quinolyl-4)-1 propanol-1(R) ou un sel de ce composé avec un acide pharmaceutiquement acceptable.

12. Médicaments selon l'une quelconque des revendications 1 à 11 sous forme de doses unitaires contenant 10 à 100 mg de substance active.

13. Composés, racémiques, énantiomères ou stéréoisomères, répondant à la formule :

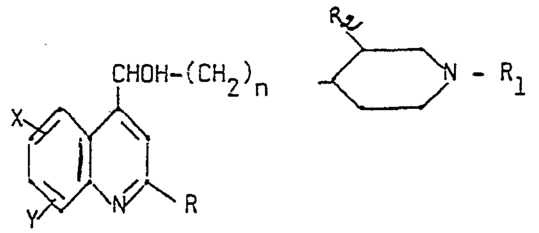

(Ia)

dans laquelle n est égal à 1, X et Y qui sont identiques ou différents, sont fixés en position 5, 6, 7 ou 8 sur le cycle de la quinoléine et représentent chacun l'atome d'hydrogène ou un groupe alcoxy comportant 1 à 3 atomes de carbone, R représente l'atome d'hydrogène, un groupe cycloalkyle ayant 3 à 8 atomes de carbone, un groupe alkyle comportant 1 à 4 atomes de carbone, un groupe phényle ou un groupe phényle substitué par un groupe alcoxy ayant 1 à 4 atomes de carbone, $R_1$ représente l'atome d'hydrogène, un groupe alkyle comportant 1 à 4 atomes de carbone ou un groupe phénylalkyle dont la partie alkyle comporte 1 à 3 atomes de carbone, $R_2$ représente l'atome d'hydrogène, un groupe alkyle comportant 1 ou 2 atomes de carbone ou un groupe alcényle comportant 2 à 4 atomes de carbone.

14. Composés selon la revendication 13, caractérisés en ce que Y est un atome d'hydrogène, X est un atome d'hydrogène ou un groupe méthoxy, R est un atome d'hydrogène ou un groupe phényle ou tertiobutyle, $R_1$ est un atome d'hydrogène et $R_2$ est un atome d'hydrogène ou un groupe éthyle ou éthényle.

15. Composés, racémiques, énantiomères ou stéréoisomères, répondant à la formule :

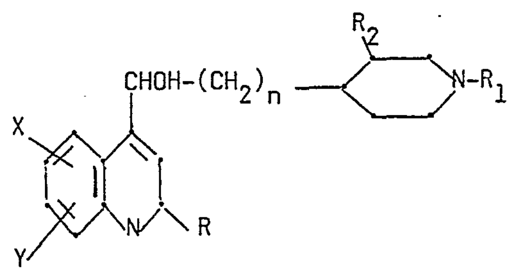

(Ib)

dans laquelle n est égal à 2, X et Y qui sont identiques ou différents, sont fixés en position 5, 6, 7 ou 8 sur le cycle de la quinoléine et représentent chacun l'atome d'hydrogène ou un groupe alcoxy comportant 1 à 3 atomes de carbone et

a) soit $R_2$ est un groupe alcényle comportant 2 à 4 atomes de carbone, R représente l'atome d'hydrogène et $R_1$ représente un groupe phénylalkyle dont la partie alkyle comporte 1 à 3 atomes de carbone,

b) soit $R_2$ est un groupe alcényle comportant 2 à 4 atomes de carbone, R représente un groupe cycloalkyle ayant 3 à 8 atomes de carbone, un groupe alkyle comportant 1 à 4 atomes de carbone, un groupe phényle éventuellement substitué par un groupe alcoxy ayant 1 à 4 atomes de carbone et $R_1$ représente l'atome d'hydrogène, un groupe alkyle comportant 1 à 4 atomes de carbone ou un groupe phénylalkyle dont la partie alkyle comporte 1 à 3 atomes de carbone,

c) soit $R_2$ est un groupe alkyle comportant 1 ou 2 atomes de carbone, R représente l'atome d'hydrogène et $R_1$ représente un groupe phénylalkyle dont la partie alkyle comporte 1 à 3 atomes de carbone,

d) soit $R_2$ représente un groupe alkyle comportant 1 ou 2 atomes de carbone, R représente un groupe alkyle comportant 1 à 4 atomes de carbone et $R_1$ représente un groupe phénylalkyle dont la partie alkyle comporte 1 à 3 atomes de carbone,

e) soit $R_2$ est un groupe alkyle comportant 1 ou 2 atomes de carbone, R représente un groupe cycloalkyle ayant 3 à 8 atomes de carbone, un groupe phényle éventuellement substitué par un groupe alcoxy ayant 1 à 4 atomes de carbone, $R_1$ représente un atome d'hydrogène, un groupe alkyle comportant 1 à 4 atomes de carbone ou un groupe phénylalkyle dont la partie alkyle comporte 1 à 3 atomes de carbone.

16. Composé selon la revendication 15, caractérisés en ce que Y est un atome d'hydrogène, X est un atome d'hydrogène ou un groupe méthoxy, R est un groupe phényle ou tertiobutyle, $R_1$ est un atome d'hydrogène et $R_2$ est un atome d'hydrogène ou un groupe éthyle ou éthényle.

17. Le composé [(diméthyl-1,1 éthyl)-2 quinolyl-4]-1 (pipéridyl-4)-3 propanol-1 (racémique).

18. Composés, racémiques ou énantiomères, répondant à la formule :

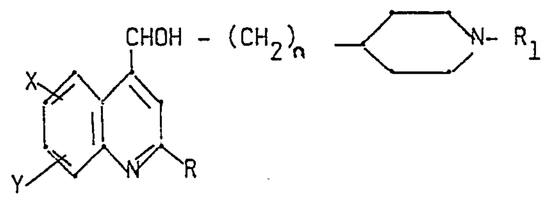

(Ic)

dans laquelle n est égal à 2, X et Y qui sont identiques ou différents, sont fixés en position 5, 6, 7 ou 8 sur le cycle de la quinoléine et représentent chacun l'atome d'hydrogène ou un groupe alcoxy comportant 1 à 3 atomes de carbone, R représente l'atome d'hydrogène, un groupe cycloalkyle ayant 3 à 8 atomes de carbone, un groupe alkyle comportant 1 à 4 atomes de carbone, un groupe phényle ou un groupe phényle substitué par un groupe alcoxy ayant 1 à 4 atomes de carbone, $R_1$ représente l'atome d'hydrogène, un groupe alkyle comportant 1 à 4 atomes de carbone ou un groupe phénylalkyle dont la partie alkyle comporte 1 à 3 atomes de carbone.

19. Composés selon la revendication 18, caractérisés en ce que Y est un atome d'hydrogène, X est un atome d'hydrogène ou un groupe méthoxy, R est un atome d'hydrogène ou un groupe phényle ou tertiobutyle et $R_1$ est un atome d'hydrogène.

20. Procédé de préparation des composés selon l'une quelconque des revendications 13, 15 et 18 caractérisé en ce que l'on réduit une cétone de formule :

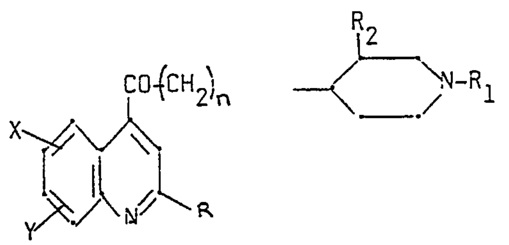

(II)

dans laquelle

a) soit n, X, Y, R, $R_1$ et $R_2$ ont les mêmes significations que dans la formule (Ia) de la revendication 13,

b) soit n, X, Y, R, $R_1$ et $R_2$ ont les mêmes significations que dans la formule (Ib) de la revendication 15,

c) soit n, X, Y, R, $R_1$ et $R_2$ ont les mêmes significations que dans la formule (Ic) de la revendication 18.

21. Procédé selon la revendication 20, caractérisé en ce que la réduction est effectuée au moyen d'un hydrure métallique réducteur.

22. Procédé selon la revendication 21, caractérisé en ce que la réduction est effectuée en présence d'un composé optiquement actif capable de complexer l'hydrure métallique réducteur utilisé.

23. Procédé de préparation des composés selon l'une quelconque des revendications 13 et 15 pour lesquels $R_2$ est un groupe alkyle ayant 2 atomes de carbone et $R_1$ n'est pas un groupe benzyle, caractérisé en ce que l'on hydrogène catalytiquement les cétones de formule (II) selon la revendication 20 pour lesquelles $R_2$ est un groupe alcényle ayant deux atomes de carbone et $R_1$ n'est pas un groupe benzyle.

24. Procédé de préparation des composés selon l'une quelconque des revendications 13, 15 et 18 pour lesquels $R_1$ est un groupe alkyle ou phénylalkyle, caractérisé en ce que l'on fait agir un agent alkylant sur les composés selon l'une quelconque des revendications 13, 15 et 18 pour lesquels $R_1$ est un atome d'hydrogène.

25. Procédé de préparation des composés selon l'une quelconque des revendications 13, 15 et 18 pour lesquels $R_1$ est $CH_3$, caractérisé en ce que l'on fait agir, en présence d'un agent réducteur, le formaldéhyde sur les composés selon l'une quelconque des revendications 13, 15 et 18 pour lesquels $R_1$ est un atome d'hydrogène.

26. Procédé selon la revendication 25, caractérisé en ce que la réaction est effectuée à une température supérieure à 50 °C.

## Claims

1. Drugs containing an active substance and a pharmaceutically acceptable vehicle, characterised in that the active substance is a compound corresponding to the formula :

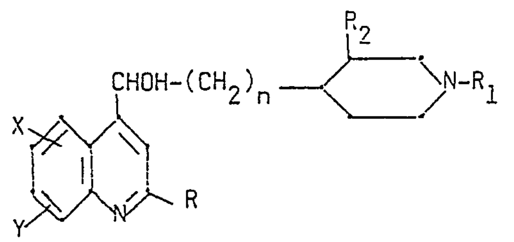

(I)

in which

a) either n equals 1, X and Y, which are identical or different, are bound in position 5, 6, 7 or 8 on the quinoline ring-system and each denote a hydrogen atom or an alkoxy group containing 1 to 3 carbon atoms, R denotes a hydrogen atom, a cycloalkyl group having 3 to 8 carbon atoms, an alkyl group containing 1 to 4 carbon atoms, a phenyl group or a phenyl group substituted with an alkoxy group having 1 to 4 carbon atoms, $R_1$ denotes a hydrogen atom, an alkyl group containing 1 to 4 carbon atoms or a phenylalkyl group in which the alkyl portion contains 1 to 3 carbon atoms, $R_2$ denotes a hydrogen atom, an alkyl group containing 1 or 2 carbon atoms or an alkenyl group containing 2 to 4 carbon atoms,

b) or n equals 2, $R_2$ denotes a hydrogen atom or an alkenyl group containing 2 to 4 carbon atoms, X, Y, R and $R_1$ have the meanings stated above in a),

c) or n equals 2, $R_2$ denotes an alkyl group containing 1 or 2 carbon atoms, R denotes a cycloalkyl group having 3 to 8 carbon atoms, a phenyl group or a phenyl group substituted with an alkoxy group having 1 to 4 carbon atoms, and X, Y and $R_1$ have the meanings stated above in a),

d) or n equals 2, $R_2$ denotes an alkyl group containing 1 or 2 carbon atoms, R denotes a hydrogen atom, $R_1$ denotes a hydrogen atom or a phenylalkyl group in which the alkyl portion contains 1 to 3 carbon atoms, and X and Y have the meanings stated above in a),

e) or n equals 2, $R_2$ denotes an alkyl group containing 1 or 2 carbon atoms, R denotes an alkyl group containing 1 to 4 carbon atoms, $R_1$ denotes a phenylalkyl group in which the alkyl portion contains 1 to 3 carbon atoms, and X and Y have the meanings stated above in a),

or a mixture of stereoisomeric compounds corresponding to the formula (I) or a salt of such a compound or mixture of stereoisomeric compounds with a pharmaceutically acceptable acid.

2. Drugs according to Claim 1, characterised in that, in the formula (I), Y is a hydrogen atom, X is a hydrogen atom or a methoxy group, R is a hydrogen atom or a phenyl or tert-butyl group, $R_1$ is a hydrogen atom and $R_2$ is a hydrogen atom or an ethyl or ethenyl group.

3. Drugs according to Claim 1, characterised in that, in the formula (I), n equals 1.

4. Drugs according to Claim 1, characterised in that, in the formula (I), R is a cycloalkyl group having 3 to 8 carbon atoms, an alkyl group having 1 to 4 carbon atoms, a phenyl group or a phenyl group substituted with an alkoxy group having 1 to 4 carbon atoms.

5. Drugs according to claim 1, characterised in that, in the formula (I), $R_2$ is a hydrogen atom.

6. Drugs according to Claim 2, characterised in that the active substance is a mixture of 3-[3(R) ethenyl-4(R)-piperidyl]-1-(6-methoxy-4-quinolyl)-1(R)-propanol and 3-[(R)-ethenyl-4(R)-piperidyl]-1-(6-methoxy-4-quinolyl)-1(S)-propanol or a salt of such a mixture with a pharmaceutically acceptable acid.

7. Drugs according to Claim 2, characterised in that the active substance is 3-[3(R)-ethenyl-4(R)-piperidyl]-1-(6-methoxy-4-quinolyl)-1(S)-propanol or a salt of this compound with a pharmaceutically acceptable acid.

8. Drugs according to Claim 2, characterised in that the active substance is 3-[3(R)-ethenyl-4(R)-piperidyl]-1-(6-methoxy-4-quinolyl)-1(R)-propanol or a salt of this compound with a pharmaceutically acceptable acid.

9. Drugs according to Claim 2, characterised in that the active substance is 1-[2-(1,1,-dimethylethyl)-4-quinolyl]-3-(4-piperidyl)-1-propanol (racemic) or a salt of this product with a pharmaceutically acceptable acid.

10. Drugs according to Claim 2, characterised in that the active substance is 3-[3(R)-ethyl-4(R)-piperidyl]-1-(6-methoxy-4-quinolyl)-1(S)-propanol or a salt of this compound with a pharmaceutically acceptable acid.

11. Drugs according to Claim 2, characterised in that the active substance is 3-[3(R)-ethyl-4(R)-piperidyl]-1-(6-methoxy-4-quinolyl)-1(R)-propanol or a salt of this compound with a pharmaceutically acceptable acid.

12. Drugs according to any one of Claims 1 to 11, in the form of unit doses containing 10 to 100 mg of active substance.

13. Racemic, enantiomeric or stereoisomeric compounds corresponding to the formula :

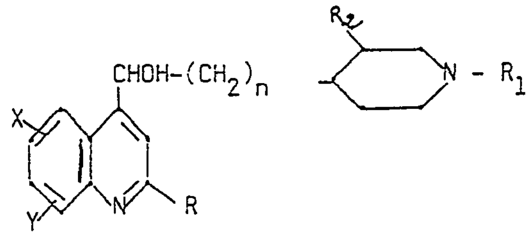

(Ia)

in which n equals 1, X and Y, which are identical or different, are bound in position 5, 6, 7 or 8 on the quinoline ring-system and each denote a hydrogen atom or an alkoxy group containing 1 to 3 carbon atoms, R denotes a hydrogen atom, a cycloalkyl group having 3 to 8 carbon atoms, an alkyl group containing 1 to 4 carbon atoms, a phenyl group or a phenyl group substituted with an alkoxy group having 1 to 4 carbon atoms, $R_1$ denotes a hydrogen atom, an alkyl group containing 1 to 4 carbon atoms or a phenylalkyl group in which the alkyl portion contains 1 to 3 carbon atoms, $R_2$ denotes a hydrogen atom, an alkyl group containing 1 or 2 carbon atoms or an alkenyl group containing 2 to 4 carbon atoms.

14. Compounds according to Claim 13, characterised in that Y is a hydrogen atom, X is a hydrogen atom or a methoxy group, R is a hydrogen atom or a phenyl or tert-butyl group, $R_1$ is a hydrogen atom and $R_2$ is a hydrogen atom or an ethyl or ethenyl group.

15. Racemic, enantiomeric or stereoisomeric compounds corresponding to the formula :

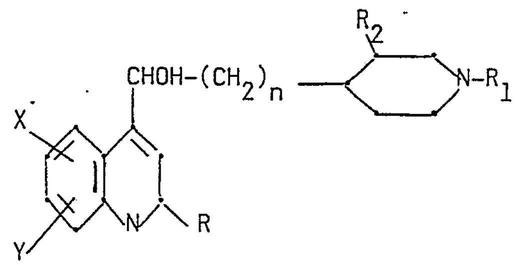

(Ib)

in which n equals 2, X and Y, which are identical or different, are bound in position 5, 6, 7 or 8 on the quinoline ring-system and each denote a hydrogen atom or an alkoxy group containing 1 to 3 carbon atoms, and

a) either $R_2$ is an alkenyl group containing 2 to 4 carbon atoms, R denotes a hydrogen atom, $R_1$ denotes a phenylalkyl group in which the alkyl portion contains 1 to 3 carbon atoms,

b) or $R_2$ is an alkenyl group containing 2 to 4 carbon atoms, R denotes a cycloalkyl group having 3 to 8 carbon atoms, an alkyl group containing 1 to 4 carbon atoms, a phenyl group optionally substituted with an alkoxy group having 1 to 4 carbon atoms, and $R_1$ denotes a hydrogen atom, an alkyl group containing 1 to 4 carbon atoms or a phenylalkyl group in which the alkyl portion contains 1 to 3 carbon atoms,

c) or $R_2$ is an alkyl group containing 1 or 2 carbon atoms, R denotes a hydrogen atom and $R_1$ denotes a phenylalkyl group in which the alkyl portion contains 1 to 3 carbon atoms,

d) or $R_2$ denotes an alkyl group containing 1 or 2 carbon atoms, R denotes an alkyl group containing 1 to 4 carbon atoms and $R_1$ denotes a phenylalkyl group in which the alkyl portion contains 1 to 3 carbon atoms,

e) or $R_2$ is an alkyl group containing 1 or 2 carbon atoms, R denotes a cycloalkyl group having 3 to 8 carbon atoms, a phenyl group optionally substituted with an alkoxy group having 1 to 4 carbon atoms, and $R_1$ denotes a hydrogen atom, an alkyl group containing 1 to 4 carbon atoms or a phenylalkyl group in which the alkyl portion contains 1 to 3 carbon atoms.

16. Compound according to Claim 15, characterised in that Y is a hydrogen atom, X is a hydrogen atom or a methoxy group, R is a phenyl or tert-butyl group, $R_1$ is a hydrogen atom and $R_2$ is a hydrogen atom or an ethyl or ethenyl group.

17. The compound 1-[2-(1,1-dimethylethyl)-4-quinolyl]-3-(4-piperidyl)-1-propanol (racemic).

18. Racemic or enantiomeric compounds corresponding to the formula :

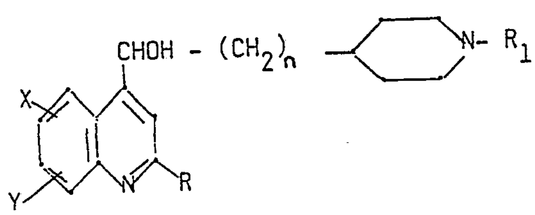

(Ic)

in which n equals 2, X and Y, which are identical or different are bound in position 5, 6, 7 or 8 on the quinoline ring-system and each denote a hydrogen atom or an alkoxy group containing 1 to 3 carbon atoms, R denotes a hydrogen atom, a cycloalkyl group having 3 to 8 carbon atoms, an alkyl group containing 1 to 4 carbon atoms, a phenyl group or a phenyl group substituted with an alkoxy group having 1 to 4 carbon atoms, $R_1$ denotes a hydrogen atom, an alkyl group containing 1 to 4 carbon atoms or a phenylalkyl group in which the alkyl portion contains 1 to 3 carbon atoms.

19. Compounds according to Claim 18, characterised in that Y is a hydrogen atom, X is a hydrogen atom or a methoxy group, R is a hydrogen atom or a phenyl or tert-butyl group and $R_1$ is a hydrogen atom.

20. Process for preparing compounds according to any one of Claims 13, 15 and 18, characterised in that the reduction is carried out of a ketone of formula

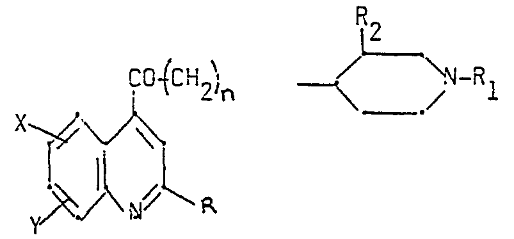

(II)

in which

a) either n, X, Y, R, $R_1$, and $R_2$ have the same meanings as in the formula (Ia) in Claim 13,

b) or n, X, Y, R, $R_1$ and $R_2$ have the same meanings as in the formula (Ib) in Claim 15,

c) or n, X, Y, R, $R_1$ and $R_2$ have the same meanings as in the formula (Ic) in Claim 18.

21. Process according to Claim 20, characterised in that the reduction is performed by means of a reducing metal hydride.

22. Process according to Claim 21, characterised in that the reduction is performed in the presence of an optically active compound capable of complexing the reducing metal hydride used.

23. Process for preparing the compounds according to any one of Claims 13 and 15, for which $R_2$ is an alkyl group having 2 carbon atoms and $R_1$ is not a benzyl group, characterised in that the ketones of formula II according to Claim 20 for which $R_2$ is an alkenyl group having 2 carbon atoms and $R_1$ is not a benzyl group are catalytically hydrogenated.

24. Process for preparing the compounds according to any one of Claims 13, 15 and 18 for which $R_1$ is an alkyl or phenylalkyl group, characterised in that an alkylating agent is caused to act on the compounds according to any one of Claims 13, 15 and 18 for which $R_1$ is a hydrogen atom.

25. Process for preparing the compounds according to any one of Claims 13, 15 and 18 for which R1 is $CH_3$, characterised in that formaldehyde is caused to act, in the presence of a reducing agent, on the compounds according to any one of Claims 13, 15 and 18 for which $R_1$ is a hydrogen atom.

26. Process according to Claim 25, characterised in that the reaction is performed at a temperature above 50 °C.

**Patentansprüche**

1. Arzneimittel enthaltend eine aktive Substanz und einen pharmazeutisch annehmbaren Träger, dadurch gekennzeichnet, daß die aktive Substanz eine Verbindung der Formel (I)

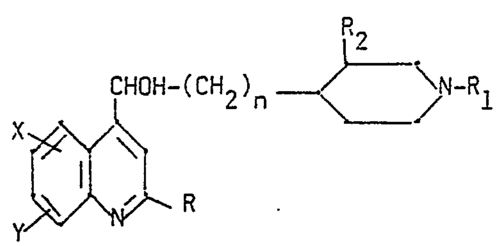

(I)

ist, worin

a) entweder n gleich 1 ist, X und Y, welche identisch oder verschieden sind, in 5-, 6-, 7- oder 8-Stellung an dem Chinolinring fixiert sind und jeweils das Wasserstoffatom oder eine Alkoxygruppe mit 1 bis 3 Kohlenstoffatomen bedeuten, R das Wasserstoffatom, eine Cycloalkylgruppe mit 3 bis 8 Kohlenstoffatomen, eine Alkylgruppe mit 1 bis 4 Kohlenstoffatomen, eine Phenylgruppe oder eine Phenylgruppe substituiert durch eine Alkoxygruppe mit 1 bis 4 Kohlenstoffatomen bedeutet, $R_1$ das Wasserstoffatom, eine Alkylgruppe mit 1 bis 4 Kohlenstoffatomen oder eine Phenylalkylgruppe bedeutet, dessen Alkylteil 1 bis 3 Kohlenstoffatome trägt, $R_2$ das Wasserstoffatom, eine Alkylgruppe mit 1 oder 2 Kohlenstoffatomen oder eine Alkenylgruppe mit 2 bis 4 Kohlenstoffatomen bedeutet,

oder

b) n = 2, $R_2$ das Wasserstoffatom oder eine Alkenylgruppe mit 2 bis 4 Kohlenstoffatomen bedeutet, X, Y, R und $R_1$ die vorstehend in a) angegebenen Bedeutungen besitzen,
oder

c) n = 2, $R_2$ eine Alkylgruppe mit 1 oder 2 Kohlenstoffatomen bedeutet, R eine Cycloalkylgruppe mit 3 bis 8 Kohlenstoffatomen, eine Phenylgruppe oder eine Phenylgruppe substituiert durch eine Alkoxygruppe mit 1 bis 4 Kohlenstoffatomen bedeutet, X, Y und $R_1$ die vorstehend in a) angegebenen Bedeutungen besitzen,
oder

d) n = 2, $R_2$ eine Alkylgruppe mit 1 oder 2 Kohlenstoffatomen bedeutet, R das Wasserstoffatom bedeutet, $R_1$ das Wasserstoffatom oder eine Phenylalkylgruppe bedeutet, deren Alkylteil 1 bis 3 Kohlenstoffatome hat, X und Y die vorstehend in a) angegebenen Bedeutungen besitzen,
oder

e) n = 2, $R_2$ eine Alkylgruppe mit 1 oder 2 Kohlenstoffatomen bedeutet, R eine Alkylgruppe mit 1 bis 4 Kohlenstoffatomen bedeutet, $R_1$ eine Phenylalkylgruppe bedeutet, deren Alkylteil 1 bis 3 Kohlenstoffatome trägt, X und Y die vorstehend in a) angegebenen Bedeutungen besitzen,
oder ein Gemisch der stereoisomeren Verbindungen der Formel (I) oder ein Salz einer solchen Verbindung oder eines Gemisches der stereoisomeren Verbindungen mit einer pharmazeutisch annehmbaren Säure.

2. Arzneimittel gemäß Anspruch 1, dadurch gekennzeichnet, daß in der Formel (I) Y ein Wasserstoffatom ist, X ein Wasserstoffatom oder eine Methoxygruppe ist, R ein Wasserstoffatom oder eine Phenyl- oder tert.-Butylgruppe ist, $R_1$ ein Wasserstoffatom und $R_2$ ein Wasserstoffatom oder eine Ethyl- oder Ethenylgruppe ist.

3. Arzneimittel gemäß Anspruch 1, dadurch gekennzeichnet, daß in der Formel (I) n gleich 1 ist.

4. Arzneimittel gemäß Anspruch 1, dadurch gekennzeichnet, daß in der Formel (I) R eine Cycloalkylgruppe mit 3 bis 8 Kohlenstoffatomen, eine Alkylgruppe mit 1 bis 4 Kohlenstoffatomen, eine Phenylgruppe oder eine Phenylgruppe substituiert durch eine Alkoxygruppe mit 1 bis 4 Kohlenstoffatomen ist.

5. Arzneimittel gemäß Anspruch 1, dadurch gekennzeichnet, daß in der Formel (I) $R_2$ ein Wasserstoffatom ist.

6. Arzneimittel gemäß Anspruch 2, dadurch gekennzeichnet, daß die aktive Substanz ein Gemisch aus 3-[3(R)-Ethenyl-4(R)-piperidyl]-1-(6-methoxy-4-chinolyl)-1(R)-propanol und 3-[3(R)-Ethenyl-4(R)-piperidyl]-1-(6-methoxy-4-chinolyl)-1(S)-propanol oder ein Salz eines solchen Gemisches mit einer pharmazeutisch annehmbaren Säure ist.

7. Arzneimittel Gemäß Anspruch 2, dadurch gekennzeichnet, daß die aktive Substanz 3-[3(R)-Ethenyl-4(R)-piperidyl]-1-(6-methoxy-4-chinolyl)-1(S)-propanol oder ein Salz dieser Verbindung mit einer pharmazeutisch annehmbaren Säure ist.

8. Arzneimittel gemäß Anspruch 2, dadurch gekennzeichnet, daß die aktive Substanz 3-[3(R)-Ethenyl-4(R)-piperidyl]-1-(6-methoxy-4-chinolyl)-1(R)-propanol oder ein Salz dieser Verbindung mit einer pharmazeutisch annehmbaren Säure ist.

9. Arzneimittel gemäß Anspruch 2, dadurch gekennzeichnet, daß die aktive Substanz 1-[2-(1,1-Dimethyl-ethyl)-4-chinolyl]-3-(4-piperidyl)-1-propanol racemisch oder ein Salz dieser Verbindung mit einer pharmazeutisch annehmbaren Säure ist.

10. Arzneimittel gemäß Anspruch 2, dadurch gekennzeichnet, daß die aktive Substanz 3-[3(R)-ethyl-4(R)-piperidyl]-1-(6-methoxy-4-chinolyl)-1(S)-propanol oder ein Salz dieser Verbindung mit einer pharmazeutisch annehmbaren Säure ist.

11. Arzneimittel gemäß Anspruch 2, dadurch gekennzeichnet, daß die aktive Substanz 3-[3(R)-Ethyl-4(R)-piperidyl]-1-(6-methoxy-4-chinolyl)-1(R)-propanol oder ein Salz dieser Verbindung mit einer pharmazeutisch annehmbaren Säure ist.

12. Arzneimittel gemäß einem der Ansprüche 1 bis 11 in Form von Einheitsdosierungen enthaltend 10 bis 100 mg aktive Substanz.

13. Racemische, enantiomere oder stereoisomere Verbindungen der Formel (Ia)

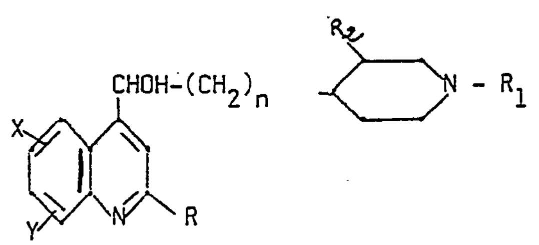

(Ia)

worin n gleich 1, X und Y, welche identisch oder verschieden sind, in 5-, 6-, 7- oder 8-Stellung am Chinolinring fixiert sind und jeweils das Wasserstoffatom oder eine Alkoxygruppe mit 1 bis 3 Kohlenstoffatomen bedeuten, R das Wasserstoffatom, eine Cycloalkylgruppe mit 3 bis 8 Kohlenstoffatomen, eine Alkylgruppe mit 1 bis 4 Kohlenstoffatomen, eine Phenylgruppe oder eine Phenylgruppe substituiert durch eine Alkoxygruppe mit 1 bis 4 Kohlenstoffatomen bedeutet, $R_1$ das Wasserstoffatom,

eine Alkylgruppe mit 1 bis 4 Kohlenstoffatomen oder eine Phenylalkylgruppe bedeutet, deren Alkylteil 1 bis 3 Kohlenstoffatome trägt, $R_2$ das Wasserstoffatom, eine Alkylgruppe mit 1 oder 2 Kohlenstoffatomen oder eine Alkenylgruppe mit 2 bis 4 Kohlenstoffatomen bedeutet.

14. Verbindungen gemäß Anspruch 13, dadurch gekennzeichnet, daß Y ein Wasserstoffatom ist, X ein Wasserstoffatom oder eine Methoxygruppe ist, R ein Wasserstoffatom oder eine Phenyl- oder tert.-Butylgruppe ist, $R_1$ ein Wasserstoffatom ist und $R_2$ ein Wasserstoffatom oder eine Ethyl- oder Ethenylgruppe ist.

15. Racemische, enantiomere oder stereoisomere Verbindungen der Formel (Ib)

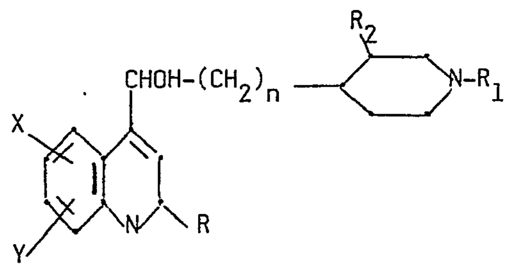

(Ib)

worin n gleich 2, X und Y, die identisch oder verschieden sind, in 5-, 6-, 7- oder 8-Stellung am Chinolinring fixiert sind und jeweils das Wasserstoffatom oder eine Alkoxygruppe mit 1 bis 3 Kohlenstoffatomen bedeuten und

a) entweder $R_2$ eine Alkenylgruppe mit 2 bis 4 Kohlenstoffatomen ist, R das Wasserstoffatom bedeutet und $R_1$ eine Phenylalkylgruppe bedeutet, deren Alkylteil 1 bis 3 Kohlenstoffatome trägt, oder

b) $R_2$ eine Alkenylgruppe mit 2 bis 4 Kohlenstoffatomen ist, R eine Cycloalkylgruppe mit 3 bis 8 Kohlenstoffatomen, eine Alkylgruppe mit 1 bis 4 Kohlenstoffatomen, eine Phenylgruppe, gegebenenfalls substituiert durch eine Alkoxygruppe mit 1 bis 4 Kohlenstoffatomen bedeutet und $R_1$ das Wasserstoffatom, eine Alkylgruppe mit 1 bis 4 Kohlenstoffatomen oder eine Phenylalkylgruppe bedeutet, deren Alkylteil 1 bis 3 Kohlenstoffatome trägt,

oder

c) $R_2$ eine Alkylgruppe mit 1 oder 2 Kohlenstoffatomen ist, R das Wasserstoffatom bedeutet und $R_1$ eine Phenylalkylgruppe bedeutet deren Alkylteil 1 bis 3 Kohlenstoffatome trägt,

oder

d) $R_2$ eine Alkylgruppe mit 1 oder 2 Kohlenstoffatomen bedeutet, R eine Alkylgruppe mit 1 bis 4 Kohlenstoffatomen bedeutet und $R_1$ eine Phenylalkylgruppe bedeutet, deren Alkylteil 1 bis 3 Kohlenstoffatome trägt,

oder

e) $R_2$ eine Alkylgruppe mit 1 oder 2 Kohlenstoffatomen ist, R eine Cycloalkylgruppe mit 3 bis 8 Kohlenstoffatomen, eine Phenylgruppe, die gegebenenfalls durch eine Alkoxygruppe mit 1 bis 4 Kohlenstoffatomen substituiert ist, bedeutet, $R_1$ ein Wasserstoffatom, eine Alkylgruppe mit 1 bis 4 Kohlenstoffatomen oder eine Phenylalkylgruppe, deren Alkylteil 1 bis 3 Kohlenstoffatome trägt, bedeutet.

16. Verbindung gemäß Anspruch 15, dadurch gekennzeichnet, daß Y ein Wasserstoffatom ist, X ein Wasserstoffatom oder eine Methoxygruppe ist, R eine Phenyl- oder tert.-Butylgruppe ist, $R_1$ ein Wasserstoffatom und $R_2$ ein Wasserstoffatom oder eine Ethyl- oder Ethenylgruppe ist.

17. Die Verbindung 1-[2-(1,1-Dimethyl-ethyl)-4-chinolyl]-3-(4-piperidyl)-1-propanol(racemisch).

18. Racemische oder enantiomere Verbindungen entsprechend der Formel (Ic)

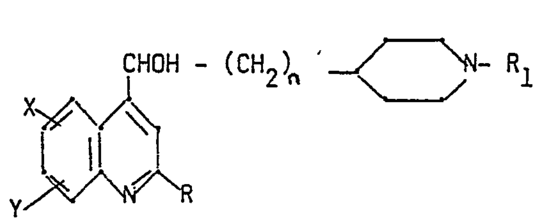

(Ic)

worin n = 2, X und Y, welche identisch oder verschieden sind, in 5-, 6-, 7- oder 8-Stellung am Chinolinring fixiert sind und jeweils das Wasserstoffatom oder eine Alkoxygruppe mit 1 bis 3 Kohlenstoffatomen bedeuten, R das Wasserstoffatom, eine Cycloalkylgruppe mit 3 bis 8 Kohlenstoffatomen, eine Alkylgruppe mit 1 bis 4 Kohlenstoffatomen, eine Phenylgruppe oder eine Phenylgruppe substituiert durch eine Alkoxygruppe mit 1 bis 4 Kohlenstoffatomen bedeutet, $R_1$ das Wasserstoffatom, eine Alkylgruppe mit 1 bis 4 Kohlenstoffatomen oder eine Phenylalkylgruppe bedeutet, deren Alkylteil 1 bis 3 Kohlenstoffatome trägt.

19. Verbindungen gemäß Anspruch 18, dadurch gekennzeichnet, daß Y ein Wasserstoffatom ist, X ein Wasserstoffatom oder eine Methoxygruppe ist, R ein Wasserstoffatom oder eine Phenyl- oder tert.-Butylgruppe ist und $R_1$ ein Wasserstoffatom ist.

20. Verfahren zur Herstellung der Verbindungen gemäß einem der Ansprüche 13, 15 und 18, dadurch gekennzeichnet, daß man ein Keton der Formel (II)

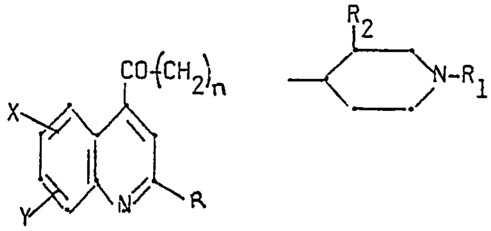

(II)

reduziert, worin

a) entweder n, X, Y, R, $R_1$ und $R_2$ dieselben Bedeutungen wie in Formel (Ia) von Anspruch 13 haben,

oder

b) n, X, Y, R, $R_1$ und $R_2$ dieselben Bedeutungen wie in Formel (Ib) von Anspruch 15 haben,

oder

c) n, X, Y, R, $R_1$ und $R_2$ dieselben Bedeutungen wie in Formel (Ic) von Anspruch 18 haben.

21. Verfahren gemäß Anspruch 20, dadurch gekennzeichnet, daß die Reduktion mittels eines reduzierenden Metallhydrids durchgeführt wird.

22. Verfahren gemäß Anspruch 21, dadurch gekennzeichnet, daß die Reduktion in Gegenwart einer optisch aktiven Verbindung, die das verwendete reduzierende Metallhydrid in einen Komplex überführen kann, durchgeführt wird.

23. Verfahren zur Herstellung von Verbindungen gemäß einem der Ansprüche 13 und 15, wobei $R_2$ eine Alkylgruppe mit 2 Kohlenstoffatomen ist und $R_1$ nicht eine Benzylgruppe ist, dadurch gekennzeichnet, daß man die Ketone der Formel (II) gemäß Anspruch 20, für die $R_2$ eine Alkenylgruppe mit 2 Kohlenstoffatomen ist und $R_1$ nicht eine Benzylgruppe ist, katalytisch hydriert.

24. Verfahren zur Herstellung der Verbindungen gemäß einem der Ansprüche 13, 15 und 18, wobei $R_1$ eine Alkyl- oder Phenylalkylgruppe ist, dadurch gekennzeichnet, daß man ein Alkylierungsmittel auf die Verbindungen gemäß einem der Ansprüche 13, 15 und 18, bei denen $R_1$ ein Wasserstoffatom ist, einwirken läßt.

25. Verfahren zur Herstellung der Verbindungen gemäß einem der Ansprüche 13, 15 und 18, für die $R_1$ Methyl darstellt, dadurch gekennzeichnet, daß man in Gegenwart eines reduzierenden Mittels Formaldehyd auf die Verbindungen gemäß einem der Ansprüche 13, 15 und 18, für die $R_1$ ein Wasserstoffatom ist, einwirken läßt.

26. Verfahren gemäß Anspruch 25, dadurch gekennzeichnet, daß die Reaktion bei einer Temperatur oberhalb 50 °C bewirkt wird.